# EUROPEAN PATENT SPECIFICATION

(11) **EP 3 423 832 B1**
(45) Date of publication and mention of the grant of the patent: **09.12.2020**
(21) Application number: 17708502.4
(22) Date of filing: 03.03.2017
(51) Int. Cl.: G01N 33/574, G01N 33/68

(54) **PROSTATE CANCER DIAGNOSTIC METHOD AND MEANS**
PROSTATAKREBS-DIAGNOSEVERFAHREN UND -MITTEL
PROCÉDÉ DE DIAGNOSTIC DU CANCER DE LA PROSTATE ET MOYENS ASSOCIÉS

(30) Priority: 04.03.2016 EP 16158770
(43) Date of publication of application: 09.01.2019
(73) Proprietor: AIT Austrian Institute of Technology GmbH, 1210 Wien (AT)
(72) Inventor: LUNA, Johana Andrea, 6162 Mutters (AT); VIERLINGER, Klemens, 1090 Vienna (AT); NOEHAMMER, Christa, 3420 Kritzendorf (AT); SOLDO, Regina, 4663 Laakirchen (AT); GAMPERL, Magdalena, 1030 Vienna (AT); MILCHRAM, Lisa, 7210 Mattersburg (AT); WEINHÄUSEL, Andreas, 7311 Neckenmarkt (AT)
(74) Representative: Sonn & Partner Patentanwälte
(86) International application number: PCT/EP2017/054979
(87) International publication number: WO 2017/149106

(56) References cited:
- EP-A2- 2 000 543
- WO-A1-2009/138392
- WO-A2-02/081638
- WO-A2-2006/119155
- US-A1- 2007 099 209
- Genecards Human Gene Database: "GOLM1 Gene - GeneCards | GOLM1 Protein | GOLM1 Antibody", , 24 August 2016 (2016-08-24), XP055297226, Retrieved from the Internet: URL:http://www.genecards.org/cgi-bin/cardd isp.pl?gene=GOLM1&keywords=golm1 [retrieved on 2016-08-24]
- Anonymous: "ProtoArray Human Protein Microarray - Thermo Fisher Scientific", , 13 December 2015 (2015-12-13), XP055413315, Retrieved from the Internet: URL:https://web.archive.org/web/2015121303 1724/https://www.thermofisher.com/order/ca talog/product/PAH0525101 [retrieved on 2017-10-06]

## Description

The present invention discloses a method of diagnosing prostate cancer by using specific markers from a set, having diagnostic power for prostate cancer diagnosis and distinguishing prostate cancer in diverse samples.

Neoplasms and cancer are abnormal growths of cells. Cancer cells rapidly reproduce despite restriction of space, nutrients shared by other cells, or signals sent from the body to stop reproduction. Cancer cells are often shaped differently from healthy cells, do not function properly, and can spread into many areas of the body. Abnormal growths of tissue, called tumours, are clusters of cells that are capable of growing and dividing uncontrollably. Tumours can be benign (noncancerous) or malignant (cancerous). Benign tumours tend to grow slowly and do not spread. Malignant tumours can grow rapidly, invade and destroy nearby normal tissues, and spread throughout the body. Malignant cancers can be both locally invasive and metastatic. Locally invasive cancers can invade the tissues surrounding it by sending out "fingers" of cancerous cells into the normal tissue. Metastatic cancers can send cells into other tissues in the body, which may be distant from the original tumour. Cancers are classified according to the kind of fluid or tissue from which they originate, or according to the location in the body where they first developed. All of these parameters can effectively have an influence on the cancer characteristics, development and progression and subsequently also cancer treatment. Therefore, reliable methods to classify a cancer state or cancer type, taking diverse parameters into consideration is desired.

In cancer-patients serum-antibody profiles change, as well as autoantibodies against the cancerous tissue are generated. Those profile-changes are highly potential of tumour associated antigens as markers for early diagnosis of cancer. The immunogenicity of tumour associated antigens is conferred to mutated amino acid sequences, which expose an altered non-self-epitope. Other explanations for its immunogenicity include alternative splicing, expression of embryonic proteins in adulthood, deregulation of apoptotic or necrotic processes and abnormal cellular localizations (e.g. nuclear proteins being secreted). Other explanations are also implicated of this immunogenicity, including alternative splicing, expression of embryonic proteins in adulthood, deregulation of apoptotic or necrotic processes, abnormal cellular localizations (e.g. nuclear proteins being secreted). Examples of epitopes of the tumour-restricted antigens, encoded by intron sequences (i.e. partially unspliced RNA were translated) have been shown to make the tumour associated antigen highly immunogenic. However, until today technical prerequisites performing an efficient marker screen were lacking.

WO 02/081638 A2 and US 2007/099209 A1 relate to nucleic acid protein expression profiles in prostate cancer. WO 2009/138392 A described peptide markers in prostate cancer. EP 2000543 A2 relates to genetic expression profiling in prostate cancer.

WO 2006/119155 discloses methods and kits for detecting serum antibodies from prostate cancer patients.

ProtoArray® Human Protein Microarray, Thermo Fisher Scientific, is a protein microarray that enables rapid profiling of thousands of biochemical interactions. It comprises over 9000 human proteins.

An object of the present invention is therefore to provide improved markers and the diagnostic use thereof for the treatment of prostate carcinoma.

The provision of specific markers permits a reliable diagnosis and stratification of patients with prostate carcinoma, in particular by means of a protein biochip.

The invention therefore relates to a method of diagnosing prostate cancer or the risk of prostate cancer in a patient by detecting antibodies against the following marker proteins or a selection of at least 2 of the marker proteins or at least 20 % of the marker proteins selected from OXA1L, GOLM1, NRXN2, PAPSS1, GNAI2, FTSJD2, CERS1, FNTB, MYO19, ADCK3, SDHA, FAM184A (List 1) in a patient, wherein the selection comprises at least OXA1L, comprising the step of detecting antibodies binding said marker proteins in a sample of the patient. The invention further relates to a method of diagnosing prostate cancer or the risk of prostate cancer in a patient by detecting antibodies against at least 2 of the marker proteins or at least 20 % of the marker proteins selected from the markers of any one of List 2, 3, 4 or any combination thereof in a patient, wherein at the markers com-prise at least OXA1L, comprising the step of detecting antibodies binding said marker proteins in a sample of the patient. Also disclosed is the use of marker proteins for the diagnosis of prostate carcinoma, wherein at least one marker protein is selected from the marker proteins of List 4 or any other marker list presented herein. The markers of List 4 are (identified by Genesymbol): OXA1L, GOLM1, NRXN2, PAPSS1, GNAI2, FTSJD2, CERS1, FNTB, MYO19, ADCK3, DHCR24, TUBGCP2, LRFN5, PSA, ATAT1, SH3BGRL, LARP1, NPC2 (includes EG:10577), UNK, ATRX, PSMA7, LCMT1, VPS37D, MITD1, CRYGD, AKR1B1, PRKAR1B, ALKBH2, CCL2, GNAI2, MTF2 (includes EG:17765), RHOG, ARMCX1, LSM12 (includes EG:124801), WDR1, RSBN1L, LAMB2, DEDD2, NEUROD6, KRT8, STX6, MDFI, FBXW5, CYHR1, MGEA5, FAHD2B, EDC4, PSD, RPL36A, ZNF238, PIK3IP1, PPIA, PRKD2, DCP1A, LCAT, MYO1F, GSTM3, PRIC285, CRABP2, CCDC136, CSF1R, ARHGAP25, IDH2, NPM1, PAF1 (includes EG:361531), HNRPDL, COPZ1, PSMC3, PRDM8, ZNF514, UBR4, WDR73, RHOB, C19orf25, MMP14, LTBP3, NUP88, DPP9, SPSB3, TSKU, TNFAIP8L2, SYS1 (includes EG:336339), RPL37A, GSTM4, PKNOX1, DRAP1, HN1, BAG6, HSPA9, LRRC47, XRCC1 (includes EG:22594), CUX1, COPS6, NSUN5P1, PSAP, LSM14B, NCBP2, SDHA, FAM98C, MAD2L1, PPP2R1A, COL4A1, CYFIP1, PRDX5, FAM220A, RPS7, EZR, EXOSC8, FAM20C, SRA1, ETS2, SLA, SERPINA1, LARS, SLIT1, FHL1 (includes EG:14199), PTPRA, ELAVL3, BBIP1, HNRNPH1, PLXNA1, PPP2R1A, IVNS1ABP, PRDX1, THOC3, PELI1, PHF2, OCIAD2, PAK6, FIS1 (includes EG:288584), IL16, IDH1, SRSF1, PABPC1, C8orf33, ARHGEF18, ACTR1B, ANKS3, ZC3H12A, PCBP1, LCK, SRM, STMN4, EPC1, NLRP1, PTOV1, C12orf51, WDR1, TCF19, ZXDC, VARS, HTATIP2, PCM1, ATCAY, PRDX3, NSD1, DUS1L, GABARAP, FAM21A/FAM21C, SPRY1, ADAR, KNDC1, HMGN2, AHCTF1, NFKB1, DCHS1, CARHSP1, CORO7/CORO7-PAM16, SSR4, KIAA1109, ABT1, PCDH7, AXIN1, TPX2, SH2B1, RPS4Y1, AKR1C4, PAM, UNC13B, HLA-C, NUDT16L1, ZNF462, NPC2 (includes EG:10577), PUM1, EDF1, COMT, PSMB10, LSM14B, SNF8, CTSW, MTUS1, ARID5A, PSMC4, KIAA0753, SFTPB, EPS15L1, ABHD8, HK1, DNM2, WASL, VPS18, ASF1B, VAV2, PPAP2B, HDAC2, SNRPD3, MICU1, C1orf131, NTAN1, SCG5, REC8 (includes EG:290227), LRPPRC, PPOX, ENOl, PCDHB14, WASL, PLA2G2A, THOC3, PAFAH1B3, PTK7, SERBP1, HNRNPA1, RASGRP2, NUP88, FAM118B, TNKS1BP1, H19, NECAP2, TK1, PLBD1, CFL1, ITGA3, ZNF668, CDKN2D, RHOT2, AKT2, NARFL, PPP2R3B, ABTB1, EMILIN1, TBC1D9B, PKM, ADNP, PPP1R12A, MRC2, PPIL1, TNKS1BP1, FGB, PPIE, SRSF4, BLOC1S1, CNPY3, IRF3, WRB, TOP2B, PDXDC1, CRAT, TCERG1, CAPZB, BABAM1, HSPA5, CNOT3, EIF3C/EIF3CL, IL17RA, DUT, GIPC1, OGFR, LMTK2, BIRC2, LCP2, CDC37, FOSB, ARFRP1, GSTP1, MYH9 (includes EG:17886), MTCH1, PSMB5, HIST3H2A, PIK3R5, NCKAP5L, C9orf86, DDX39B, TINAGL1, RGS1, INPPL1, MAN2C1, PRKCZ, DDOST, EHD1, USP5, PLEC, SLC35A2, HARS, SMG8, RPL10A, ARHGDIA, C22orf46, KRBA1, NFATC3, ATP5D, COPE, SMYD4, E2F1, KDM3A, PIK3R2, CLIC1, USP28, MORF4L1, POLR2G, TRIM78P, COG4, RHOT2, TACC2, YWHAE, IP6K2, IKBKB, RPA3, AKR1B1, CACNA1E, POTEE/POTEF, KLHL23/PHOSPHO2-KLHL23, MEPCE, EIF5A, WDR1, DOCK9, PLXNB2, NR4A1, RPL4, MBD1, VCP, H19, RARA, CDH2, KIF2A, FXYD5, PPA1, EEF1G, RIC8A, ZNF12, B4GALT2, NONO, FNDC4, SMARCC2, CYR61, PPP1CA, NDUFS2, OBFC1, WASH1/WASH5P, HSPA4, PBXIP1, WASH1/WASH5P, PLCG1, HMGB2, GTF2F1, UBC, CELF3, KIF1A, KARS, RNF216, TGS1, NFIX, SGSH, PLEKHO1, TAOK2, MLL5, LAMB1, ZNF431, C17orf28, BAZ1B, UHRF2, ATP5SL, PEX7, TSC2, TMSB10/TMSB4X, HNRNPA1, LIMS2, TBC1D13, UROD, KLF4, BZW2, SULF2, HLA-E, PRRC2A, TBC1D2, H3F3A/H3F3B, GRK6, HIP1R, ARPC5L, NFKB2, SF3B2, PSMC3, ARPC1B, NEUROD2, MGA, C1orf122, SYNE2, NOA1, INPP5F, CDK5RAP3, PABPC1, MDN1, LARP4B, UBE3C, HAGH, NIN, HDAC10, RPS4Y2, GMIP, CCDC88C, ATP1B3, SPOCK2, CYFIP2, TAF1C, WDR25, BAZ1A, NFKBIA, HLA-B, TYK2, C19orf6, SERBP1, SLC25A3, QARS, PPP1R9B, DOCK2, AP2S1, DIS3L, CCNB1IP1, ZNF761, SMARCC2, MKS1 (includes EG:287612), FCHO1, TYMP, COQ6, TELO2, XPNPEP3, TXNDC11, TRIO, HIVEP3, CD44, KPNB1, PCBP2, NPEPL1, PLCB2, FBXO6, PRMT1, ATXN7L2, TADA3, MRPL38 (includes EG:303685), PTBP1, MAGED4/MAGED4B, SEC16A, SLC35B2, ADAMTS10, ZNF256, GBAS, DNMT3A, KCNJ14, PEPD, PITRM1, LSM14A, NDUFV1, TOX2, CAD, HCFC1, WDR11, POLR2J4, TOLLIP, SUGP1, CHGA, HDAC1, HSP90AB1, KLF5, SNX9, UQCRC1, GALK1, KIAA1731, HSPG2, TLN1, COPS6, TMED3, DUS2L, PPP1R9B, LOC407835, TNRC6B, PKM, DAK, VDAC1, LRP4, ULK3, PHKB, NBEA, GTF3C1, IVNS1ABP, AHCY, WDR82, HACL1, GOLGA4, USP22, KIF2A, APOBEC3A, TTC27, TMEM131, YWHAQ, SEC24B, ZNF439, HTRA1, WDTC1, LARP7, BIN3, PTPRO, GET4, SUPV3L1, TUBB2B, EEFSEC, DHX34, PDZD4, MYCBP2, BRD9, GATA1, USP39, DFFA, USP7, ATP8B3, UBE2N, C17orf28, EIF3C/EIF3CL, IMPDH1, SART3, ANXA1. The expression of any of these markers and the emergence of auto-antibodies in a patient are indicators for prostate cancer. Antibodies can be detected.

Although the detection of a single marker can be sufficient to indicate a risk for prostate cancer, it is preferred to use more than one marker, e.g. 2, 3, 4, 5, 6, 7, 8, 9, 10, 11 or 12 or more markers in combination, especially if combined with statistical analysis. Means for statistical analysis can e.g. be provided on a computer-readable memory device for operation on a computer. Such analysis means, e.g. a computer program, may be capable to analyse marker measurement data and comparison to evaluate a risk of prostate cancer. From a diagnostic point of view, a single autoantigen based diagnosis can be improved by increasing sensitivity and specificity by using a panel of markers where multiple auto-antibodies are being detected simultaneously. Auto-antibodies in a sample can be detected by binding to the marker proteins or their antigenic fragments or epitopes. Particular preferred combinations are of markers within one of the marker lists 1 to 13, 3p1, 3p2, 3p3 as identified further herein.

The inventive markers are suitable protein antigens that are overexpressed in tumours. The markers usually cause an antibody reaction in a patient. Therefore, the most convenient method to detect the presence of these markers in a patient is to detect (auto)antibodies against these marker proteins in a sample from the patient, especially a body fluid sample, such as blood, plasma or serum.

To detect an antibody in a sample it is possible to use marker proteins as binding agents and subsequently to detect bound antibodies. It is not necessary to use the entire marker proteins but it is sufficient to use antigenic fragments that are bound by the antibodies. "Antigenic fragment" herein relates to a fragment of the marker protein that causes an immune reaction against said marker protein in a human, especially a male. Preferred antigenic fragments of any one of the inventive marker proteins are the fragments of the clones as identified by the UniqueID or cloneID. Such antigenic fragments may be antigenic in a plurality of humans, such as at least 5, or at least 10 individuals.

"Diagnosis" for the purposes of this invention means the positive determination of prostate carcinoma by means of the marker proteins according to the invention as well as the assignment of the patients to prostate carcinoma. The term "diagnosis" covers medical diagnostics and examinations in this regard, in particular in-vitro diagnostics and laboratory diagnostics, likewise proteomics and peptide blotting. Further tests can be necessary to be sure and to exclude other diseases. The term "diagnosis" therefore likewise covers the differential diagnosis of prostate carcinoma by means of the marker proteins according to the invention and the risk or prognosis of prostate carcinoma.

The invention and any marker described herein can be used to distinguish between normal benign prostate hyperplasia and prostate cancer. A positive result in distinguishing said indications can prompt a further cancer test, in particular more invasive tests than a blood test such as a biopsy. Especially preferred the invention is combined with a PSA test.

The inventive markers are preferably grouped in sets of high distinctive value. Such a grouping can be according to lists 3p1, 3p2, 3p3, 5-13.

Disclosed is the method of diagnosing prostate cancer or the risk of prostate cancer in a patient by detecting at least 2, 3, 4, 5, 6 or more or any number as disclosed above, of the marker proteins selected from the markers of each List 1-13, 3p1, 3p2 or 3p2 in a patient comprising the step of detecting antibodies binding said marker proteins, detecting said marker proteins or antigenic fragments thereof in a sample of the patient. Also provided is a method of diagnosing prostate cancer or the risk of prostate cancer in a patient by detecting at least 20%, preferably at least 30%, especially preferred at least 40%, at least 50%, at least 60%, at least 70%, at least 80% at least 90% or all of the marker proteins selected from the markers of each List 1-13, 3p1, 3p2, 3p3 in a patient comprising the step of detecting antibodies binding said marker proteins, detecting said marker proteins or antigenic fragments thereof in a sample of the patient.

Especially preferred is a combination of detecting at least 2, 3, 4, 5, 6 or more of the markers of List 1, which are OXA1L, GOLM1, NRXN2, PAPSS1, GNAI2, FTSJD2, CERS1, FNTB, MYO19, ADCK3, SDHA, FAM184A, wherein the selection comprises at least OXA1L. Especially preferred, in any set for detection of the invention, markers SDHA and/or FAM184A are used. These markers proved to have the highest versatility independent of detection platform, e.g. microarray detection or ELISA. These sets allow especially good results when combined with a PSA test. In particular preferred is a combination of OXA1L und GOLM1, which can be further combined with any one or more marker of List 1, e.g. NRXN2, PAPSS1, GNAI2, FTSJD2, CERS1, FNTB, MYO19, ADCK3, SDHA, FAM184A or with any one or more of the markers of List 4.

Disclosed is a combination of detecting at least 2, 3, 4, 5, 6 or more of the markers of List 5, which are ATAT1, CCDC136, CDK5RAP3, GOLGA4, HCFC1, HLA-C, HNRNPA1, MYO19, NONO, PLEC, PPP1R9B, SNX9, SULF2, USP5, WDR1 and ZC3H12A. These markers resulted in very good prostate vs. benign classification.

Disclosed is a combination of detecting at least 2, 3, 4, 5, 6 or more of the markers of List 6, which are ARID5A, EIF3C, FCHO1, HAGH, IVNS1ABP, KLHL23, LARP7, NDUFS2, PLXNB2, SMARCC2, TOLLIP, TRIO and WDR11. These markers resulted in very good prostate vs. benign classification.

Disclosed is a combination of detecting at least 2, 3, 4, 5, 6 or more of the markers of List 7, which are AKR1C4, B4GALT2, BRD9, COPS6, EEFSEC, HCFC1, MYO1F, NBEA, NEUROD2, PPP1CA, PSMC4, RASGRP2, RPA3, SMG8, SUGP1, TMEM131 and TUBB2B. These markers resulted in very good prostate vs. benign classification.

Disclosed is a combination of detecting at least 2, 3, 4, 5, 6 or more of the markers of List 8, which are NRXN2, GNAI2, PAPSS1, CERS1, GOLM1, MYO19, ADCK3, FAM184A, FNTB, SDHA. These markers resulted in very good discriminatory power.

Disclosed is a combination of detecting at least 2, 3, 4, 5, 6 or more of the markers of List 9, which are PSMA7, PSA, NRXN2, PAPSS1, FAM20C, NUP88, PTOV1, DRAP1, ASF1B, CAPZB, PCBP1, PPP1R12A, PSMC4, LTBP3, FNTB, EDC4, SSR4, SMARCC2, LAMB2, GOLM1. These markers resulted in very good discriminatory power.

Disclosed is a combination of detecting at least 2, 3, 4, 5, 6 or more of the markers of List 10, which are PSMC4, DNMT3A, TGS1, NRXN2, GRK6, TBC1D2, ZNF431, DUS2L, MGA, LSM14. These markers resulted in very good discriminatory power.

Disclosed is a combination of detecting at least 2, 3, 4, 5, 6 or more of the markers of List 11, which are PLEC, RPL36A, HSP90AB1, UBR4, NRXN2, ABTB1, GSTP1, HARS, ARFRP1, USP5. These markers resulted in very good discriminatory power.

Disclosed is a combination of detecting at least 2, 3, 4, 5, 6 or more of the markers of List 12, which are HIST3H2A, RPS4Y2, HAGH, HNRPDL, COPZ1, CRAT, GET4, SUPV3L1, ACTR1B, UBE3C. These markers resulted in very good discriminatory power.

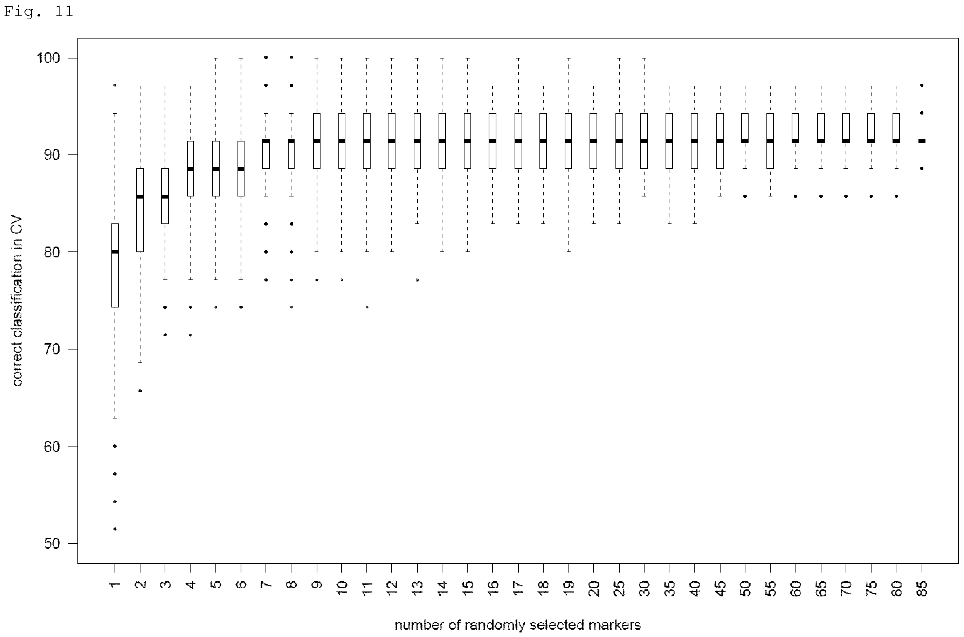

Disclosed is a combination of detecting at least 2, 3, 4, 5, 6 or more of the markers of List 13, which are PSMA7, PSA, NRXN2, PAPSS1, PLXNB2, FAM20C, TOLLIP, LSM14B, KDM3A, SYNE2. These markers resulted in very good discriminatory power. Disclosed is a combination of detecting at least 2, 3, 4, 5, 6, 7, 8, 9, 10 or more of the markers of List 3p1, which are. This list is given in the examples. List 3p1 is a part of list 3 and the markers performed remarkably well. Indeed any combination of markers of list 3p1. A random permutation analysis, i.e. repeated random picks of markers of this list showed even with low marker amounts exceptional classification rates (See Fig. 11) .

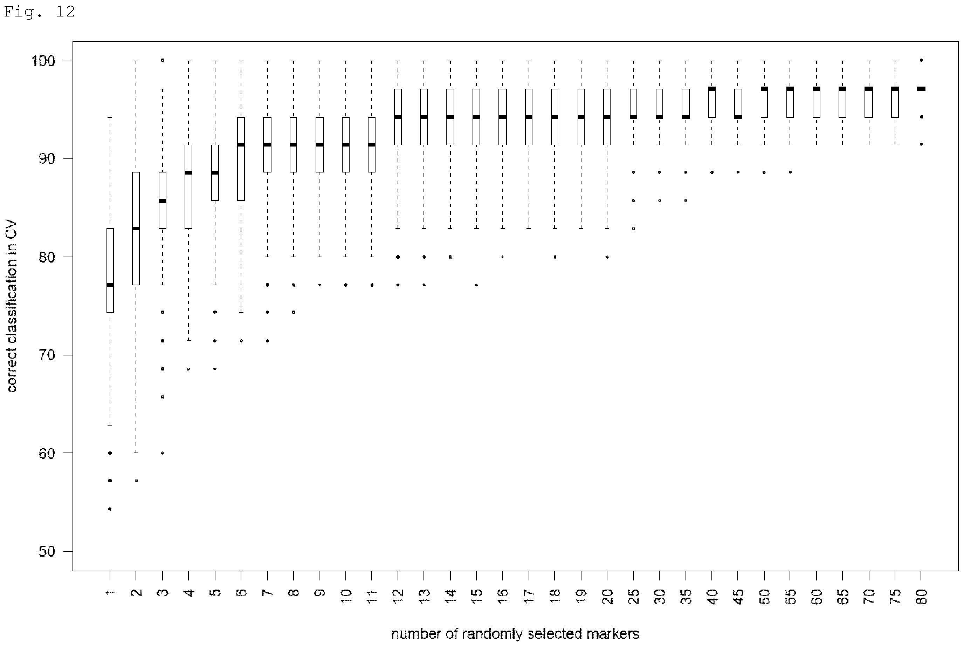

Disclosed is a combination of detecting at least 2, 3, 4, 5, 6, 7, 8, 9, 10 or more of the markers of List 3p2, which are. This list is given in the examples. List 3p2 is a part of list 3 and the markers performed remarkably well. Indeed any combination of markers of list 3p2. A random permutation analysis, i.e. repeated random picks of markers of this list showed even with low marker amounts exceptional classification rates (See Fig. 12) .

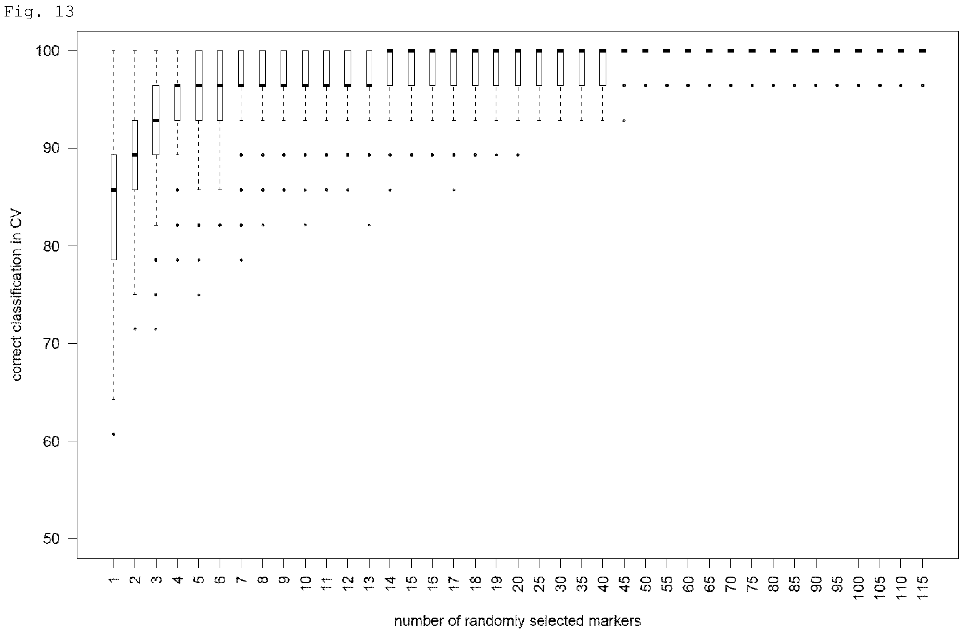

Disclosed is a combination of detecting at least 2, 3, 4, 5, 6, 7, 8, 9, 10 or more of the markers of List 3p3, which are. This list is given in the examples. List 3p3 is a part of list 3 and the markers performed remarkably well. Indeed any combination of markers of list 3p3. A random permutation analysis, i.e. repeated random picks of markers of this list showed even with low marker amounts exceptional classification rates (See Fig. 13) .

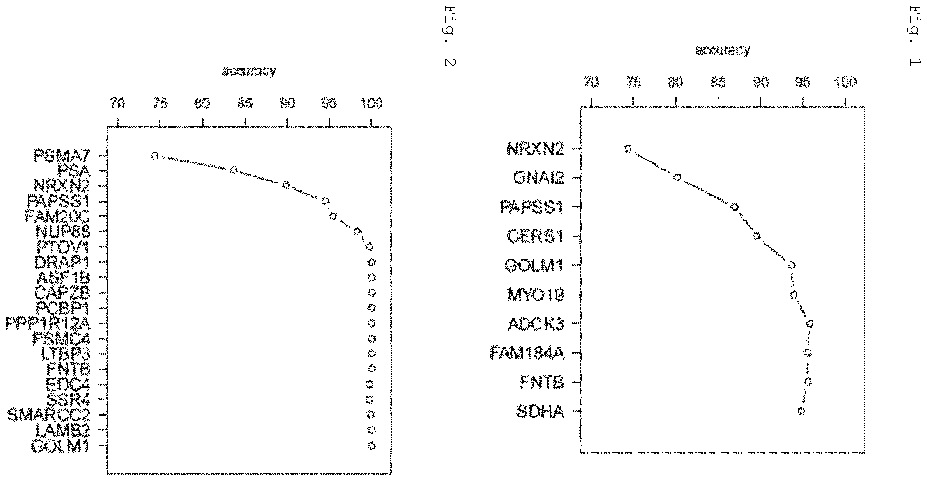

In particular preferred are the markers as shown in Figures 1 to 6, which were evaluated according to a best subset selection from the indicated list of origin. From left to right, additional markers are added to the ones on the left and each incremental marker addition substantially increases classification accuracy. Disclosed are at least 2, 3, 4, 5, 6 or more markers from any set as disclosed in any of figures 1 to 6. Preferably, the at least 2, 3, 4, 5, 6 or more markers are picked from the markers shown left to right as shown in the figures.

"Marker" or "marker proteins" are diagnostic indicators found in a patient and are detected, directly or indirectly by the inventive methods. Indirect detection is preferred. In particular, all of the inventive markers have been shown to cause the production of (auto)antigens in cancer patients or patients with a risk of developing cancer. The easiest way to detect these markers is thus to detect these (auto)antibodies in a blood or serum sample from the patient. Such antibodies can be detected by binding to their respective antigen in an assay. Such antigens are in particular the marker proteins themselves or antigenic fragments thereof. Suitable methods exist in the art to specifically detect such antibody-antigen reactions and can be used according to the invention. Preferably the entire antibody content of the sample is normalized (e.g. diluted to a pre-set concentration) and applied to the antigens. Preferably the IgG, IgM, IgD, IgA or IgE antibody fraction, is exclusively used. Preferred antibodies are IgG. Preferably the subject is a human, in particular a male.

Some markers are more preferred than others. Especially preferred markers are those which are represented at least 2, at least 3, at least 4, at least 5, at least 6, times in any one of lists 1 to 13, 3p1, 3p2, 3p3. These markers are preferably used in any one of the methods or sets.

Disclosed is a method of selecting such at least 2 markers (or more as given above) or at least 20% of the markers (or more as given above) of any one of the inventive sets with high specificity. Such a method includes comparisons of signal data for the inventive markers of any one of the inventive markers sets, especially as listed in lists 1 to 13, with said signal data being obtained from control samples of known prostate cancer conditions or indications and further statistically comparing said signal data with said conditions thereby obtaining a significant pattern of signal data capable of distinguishing the conditions of the known control samples.

In particular, the control samples may comprise one or more cancerous control (preferably at least 5, or at least 10 cancerous controls) and a healthy or non-cancerous control (preferably at least 5, or at least 10 healthy controls). Preferably 2 different indications are selected that shall be distinguished

The control samples can be used to obtain a marker dependent signal pattern as indication classifier. Such a signal pattern can be obtained by routine statistical methods, such as binary tree methods. Common statistical methods calculate a (optionally multi-dimensional) vector within the multitude of control data signal values as diagnostically significant distinguishing parameter that can be used to distinguish one or more indications from other one or more indications. The step usually comprises the step of "training" a computer software with said control data. Such pre-obtained training data or signal data can be provided on a computer-readable medium to a practitioner who performs the inventive diagnosis.

Preferably, the method comprises optimizing the selection process, e.g. by selecting alternative or additional markers and repeating said comparison with the controls signals, until a specificity and/or sensitivity of at least 75% is obtained, preferably of at least 80%, at least 85%, at least 90%, at least 95%.

Binding events can be detected as known in the art, e.g. by using labelled secondary antibodies. Such labels can be enzymatic, fluorescent, radioactive or a nucleic acid sequence tag. Such labels can also be provided on the binding means, e.g. the antigens as described in the previous paragraph. Nucleic acid sequence tags are especially preferred labels since they can be used as sequence code that not only leads to quantitative information but also to a qualitative identification of the detection means (e.g. antibody with certain specificity). Nucleic acid sequence tags can be used in known methods such as Immuno-PCR. In multiplex assays, usually qualitative information is tied to a specific location, e.g. spot on a microarray. With qualitative information provided in the label, it is not necessary to use such localized immunoassays. In is possible to perform the binding reaction of the analyte and the detection means, e.g. the serum antibody and the labelled antigen, independent of any solid supports in solution and obtain the sequence information of the detection means bound to its analyte. A binding reaction allows amplification of the nucleic acid label in a detection reaction, followed by determination of the nucleic acid sequence determination. With said determined sequence the type of detection means can be determined and hence the marker (analyte, e.g. serum antibody with tumour associated antigen specificity).

Preferably the inventive method further comprises detecting PSA in a sample from a patient comprising the step of said marker protein or antigenic fragments thereof in a sample of the patient. PSA protein can be detected according to any standard test known. The PSA blood test is the current standard for prostate cancer diagnosis, and has an accuracy of about 60-66% if used alone. Surprisingly, the accuracy can be substantially increased if combined with any other marker or list combination according to the invention. The other markers are preferably tested by detecting auto-antibodies, contrary to PSA, which is preferably tested by determining blood, plasma or serum PSA protein that is bound directly to a detection agent, like an affinity capturing agent. Both, PSA protein (see example 5 and references therein) or nucleic acids (McDermed et al., 2012, Clinical Chemistry 58(4): 732-740) can be detected in the sample. PSA protein in the sample can be detected by an affinity assay, preferably with an immobilized affinity capturing agent. An affinity capturing agent is e.g. an antibody or functional fragment thereof. Immobilization is preferably on a solid support, e.g. a microtiter well, a microarray plate or a bead. Such a PSA capturing agent and preferably also a secondary antibody to PSA with a label can be used in the inventive method or provided in the inventive kit. Nucleic acids are preferably detected by a hybridization probe, with optional amplification, especially preferred is immune-PCR.

In preferred embodiments of the invention the step of detecting antibodies binding said marker proteins, detecting said marker proteins or antigenic fragments thereof comprises comparing said detection signal with detection signals of a benign prostate hyperplasia controls and comparing said detection signals, wherein an increase in the detection signal indicates prostate cancer or said risk of prostate cancer.

In preferred embodiments of the invention the step of detecting antibodies binding said marker proteins, detecting said marker proteins or antigenic fragments thereof comprises comparing said detection signal with detection signals of a cancerous control and comparing said detection signals. In particular preferred, especially in cases of using more marker sets of 2 or more markers as mentioned above, a statistical analysis of the control is performed, wherein the controls are used to obtain a marker dependent signal pattern as indication classifier and the marker dependent signals of the sample to be analysed is compared with and/or fitted onto said pattern thereby obtaining information of the diagnosed condition or indication. Such statistical analysis is usually dependent on the used analytical platform that was used to obtain the signal data, given that signal data may vary from platform to platform. Such platforms are e.g. different microarray or solution based setups (with different labels or analytes - such as antigen fragments - for a particular marker). Thus, the statistical method can be used to calibrate each platform to obtain diagnostic information with high sensitivity and specificity. The step usually comprises the step of "training" a computer software with said control data. Alternatively, pre-obtained training data can be used. Such pre-obtained training data or signal data can be provided on a computer-readable medium to a practitioner.

In further embodiments a detection signal from the sample of a patient in amplitude of at least 60%, preferably at least 80%, of the cancerous control indicates prostate cancer or said risk of prostate cancer.

Usually not all of the inventive markers or detection agents may lead to a signal. Nevertheless, only a fraction of the signals is suitable to arrive at a diagnostic decision. In preferred embodiments of the invention a detection signal in at least 60%, preferably at least 70%, least 75%, at least 85%, or in particular preferred at least 95%, even more preferred all, of the used markers indicates prostate cancer or said risk of prostate cancer.

The present diagnostic methods further provide necessary therapeutic information to decide on a surgical intervention. Therefore, the present invention also provides a method of treating a patient comprising prostate cancer or according to any aspect or embodiment of the invention and removing said prostate cancer. "Stratification or therapy control" for the purposes of this invention means that the method according to the invention renders possible decisions for the treatment and therapy of the patient, whether it is the hospitalization of the patient, the use, effect and/or dosage of one or more drugs, a therapeutic measure or the monitoring of a course of the disease and the course of therapy or etiology or classification of a disease, e.g., into a new or existing subtype or the differentiation of diseases and the patients thereof.

One skilled in the art is familiar with expression libraries, they can be produced according to standard works, such as Sambrook et al, "Molecular Cloning, A laboratory handbook, 2nd edition (1989), CSH press, Cold Spring Harbor, N.Y. Expression libraries are also preferred which are tissue-specific (e.g., human tissue, in particular human organs). Members of such libraries can be used as inventive antigen for use as detection agent to bind analyte antibodies. Furthermore included according to the invention are expression libraries that can be obtained by exon-trapping. A synonym for expression library is expression bank. Also preferred are protein biochips or corresponding expression libraries that do not exhibit any redundancy (so-called: Uniclone(R) library) and that may be produced, for example, according to the teachings of WO 99/57311 and WO 99/57312. These preferred Uniclone libraries have a high portion of nondefective fully expressed proteins of a cDNA expression library. Within the context of this invention, the antigens can be obtained from organisms that can also be, but need not be limited to, transformed bacteria, recombinant phages, or transformed cells from mammals, insects, fungi, yeasts, or plants. The marker antigens can be fixed, spotted, or immobilized on a solid support. Alternatively, it is also possible to perform an assay in solution, such as an Immuno-PCR assay.

In a further aspect, the present invention provides a kit of diagnostic agents suitable to detect any marker or marker combination as described as invention above, wherein the marker or marker combination comprises at least OXA1L, wherein said diagnostic agents comprise marker proteins or antigenic fragments thereof suitable to bind antibodies in a sample, especially preferred wherein said diagnostic agents are immobilized on a solid support or in solution, especially when said markers are each labelled with a unique label, such as a unique nucleic acid sequence tag, the kit further comprising a computer-readable medium or a computer program product, comprising signal data for control samples with known conditions selected from prostate cancer, and/or calibration or training data for analysing said markers provided in the kit for diagnosing prostate cancer or distinguishing conditions selected from healthy conditions and prostate cancer. The inventive kit may further comprise detection agents, such as secondary antibodies, in particular anti-human antibodies, and optionally also buffers and dilution reagents. The invention therefore likewise relates to the object of providing a diagnostic device or an assay, in particular a protein biochip, ELISA or Immuno-PCR assay, which permits a diagnosis or examination for prostate carcinoma.

Additionally, the marker proteins (as binding moieties for antibody detection) can be present in the respective form of a fusion protein, which contains, for example, at least one affinity epitope or tag. The tag may be one such as contains c-myc, his tag, arg tag, FLAG, alkaline phosphatase, VS tag, T7 tag or strep tag, HAT tag, NusA, S tag, SBP tag, thioredoxin, DsbA, a fusion protein, preferably a cellulose-binding domain, green fluorescent protein, maltose-binding protein, calmodulin-binding protein, glutathione S-transferase, or lacZ, a nanoparticle or a nucleic acid sequence tag. Such a nucleic acid sequence can be e.g. DNA or RNA, preferably DNA.

In all of the embodiments, the term "solid support" covers embodiments such as a filter, a membrane, a magnetic or fluoro-phore-labeled bead, a silica wafer, glass, metal, ceramics, plastics, a chip, a target for mass spectrometry, a matrix, a bead or microtiter well. However, a filter is preferred according to the invention.

As a filter, furthermore PVDF, nitrocellulose, or nylon is preferred (e.g., Immobilon P Millipore, Protran Whatman, Hybond N+ Amersham).

In another preferred embodiment of the arrangement according to the invention, the arrangement corresponds to a grid with the dimensions of a microtiter plate (8-12 wells strips, 96 wells, 384 wells, or more), a silica wafer, a chip, a target for mass spectrometry, or a matrix.

Another method for detection of the markers is an immunosorbent assay, such as ELISA. When detecting autoantibodies, preferably the marker protein or at least an epitope containing fragment thereof, is bound to a solid support, e.g. a microtiter well. The autoantibody of a sample is bound to this antigen or fragment. Bound autoantibodies can be detected by secondary antibodies with a detectable label, e.g. a fluorescence label. The label is then used to generate a signal in dependence of binding to the autoantibodies. The secondary antibody may be an anti-human antibody if the patient is human or be directed against any other organism in dependence of the patient sample to be analysed. The kit may comprise means for such an assay, such as the solid support and preferably also the secondary antibody. Preferably the secondary antibody binds to the Fc part of the (auto) antibodies of the patient. Also possible is the addition of buffers and washing or rinsing solutions. The solid support may be coated with a blocking compound to avoid unspecific binding.

Preferably the inventive kit also comprises non-diagnostic control proteins, which can be used for signal normalization. These control proteins bind to moieties, e.g. proteins or antibodies, in the sample of a diseased patient same as in a benign prostate hyperplasia controls. In addition to the inventive marker proteins any number, but preferably at least 2 controls can be used in the method or in the kit.

Preferably the inventive kit is limited to a particular size. According to these embodiments of the invention the kit comprises at most 3000 diagnostic agents, preferably at most 2500 diagnostic agents, at most 2000 diagnostic agents, at most 1500 diagnostic agents, at most 1200 diagnostic agents, at most 1000 diagnostic agents, at most 800 diagnostic agents, at most 500 diagnostic agents, at most 300 diagnostic agents, at most 200 diagnostic agents, at most 100 diagnostic agents, such as marker proteins or antigenic fragments thereof.

In especially preferred embodiments of the invention the kit further comprises a computer-readable medium or a computer program product, such as a computer readable memory devices like a flash storage, CD-, DVD- or BR-disc or a hard drive, comprising signal data for the control samples with known conditions selected from cancer and/or of benign prostate hyperplasia controls, and/or calibration or training data for analysing said markers provided in the kit for diagnosing prostate cancer or distinguishing conditions or indications selected from benign prostate hyperplasia controls.

The kit may also comprise normalization standards, that result in a signal independent of a benign prostate hyperplasia controls condition and cancerous condition. Such normalization standards can be used to obtain background signals. Such standards may be specific for ubiquitous antibodies found in a human, such as antibodies against common bacteria such as *E. coli.* Preferably the normalization standards include positive and negative (leading to no specific signal) normalization standards.

The present invention is further illustrated by the following figures and examples, without being limited to these embodiments of the invention.

### Figures:

Fig. 1 shows the best subset selection for List 8.
Fig. 2 shows the best subset selection for List 9.
Fig. 3 shows the best subset selection for List 10.
Fig. 4 shows the best subset selection for List 11.
Fig. 5 shows the best subset selection for List 12.
Fig. 6 shows the best subset selection for List 13.
Fig. 7 shows a permutation analysis of the markers of List 1.
Fig. 8 shows a permutation analysis of the markers of List 2.
Fig. 9 shows a permutation analysis of the markers of List 3.
Fig. 10 shows a permutation analysis of the markers of List 4.
Fig. 11 shows a permutation analysis of the markers of List 3p1.
Fig. 12 shows a permutation analysis of the markers of List 3p2.
Fig. 13 shows a permutation analysis of the markers of List 3p3.

### Examples:

### Example 1: Patient samples

Biomarker screening has been performed with serum samples from a test set of serum samples derived from 49 individuals with confirmed prostate-carcinoma and 49 benign prostate hyperplasia controls (n=98). All these individuals have been elucidated either by histologically verified PCa cases (prostateosco-py) and hospital-based controls with benign prostate hyperplasia in which the presence of PCa was excluded either clinically (13/49 or 27%) or histologically (36/49 or 73%).

### Example 2: Immunoglobuline (IgG) purification from the serum or plasma samples

The patient serum or plasma samples were stored at -80 °C before they were put on ice to thaw them for IgG purification using Melon Gel 96-well Spin Plate according the manufacturer's instructions (Pierce). In short, 10µl of thawed sample was diluted in 90 µl of the equilibrated purification buffer on ice, then transferred onto Melon Gel support and incubated on a plate shaker at 500 rpm for 5 minutes. Centrifugation at 1,000 x g for 2 minutes was done to collect the purified IgG into the collection plate.

Protein concentrations of the collected IgG samples were measured by absorbance measures at 280nm using an Epoch Micro-Volume Spectrophotometer System (Biotec, USA). IgG-concentrations of all samples were concentration-adjusted and 0.4 mg/ml of samples were diluted 1:1 in PBS2x buffer with Tri-tonX 0.2% and 6% skim milk powder for microarray analyses.

### Example 3: Microarray design

A protein-chip named "16k protein chip" from 15,417 human cDNA expression clones derived from the Unipex cDNA expression library plus technical controls was generated. Using this 16k protein chip candidate markers were used to identify autoantibody profiles suitable for unequivocal distinction of prostate cancer and benign prostate hyperplasia controls.

Protein-microarray generation and processing was using the Unipex cDNA expression library for recombinant protein expression in E.coli. His-tagged recombinant proteins were purified using Ni-metal chelate chromatography and proteins were spotted in duplicates for generation of the microarray using ARChipEpoxy slides.

### Example 4: Preparation, processing and analyses of protein microarrays

The microarray with printed duplicates of the protein marker candidates was blocked with DIG Easy Hyb (Roche) in a stirred glass tank for 30 minutes. Blocked slides were washed 3x for 5 minutes with fresh PBSTritonX 0.1% washing buffer with agitation. The slides were rinsed in distilled water for 15 seconds to complete the washing step and remove leftovers from the washing buffer. Arrays were spun dry at 900rpm for 2 minutes. Microarrays were processed using the Agilent Microarray Hybridisation Chambers (Agilent) and Agilent's gasket slides filled with 490 µl of the prepared sample mixture and processed in a hybridization oven for 4h at RT with a rotation speed of 12. During this hybridization time the samples were kept under permanent rotating conditions to assure a homolog dispensation.

After the hybridization was done, the microarray slides were washed 3x with the PBSTritonX 0.1% washing buffer in the glass tank with agitation for 5 minutes and rinsed in distilled water for about 15 seconds. Then, slides were dried by centrifugation at 900 rpm for 2 minutes. IgG bound onto the features of the protein-microarrays were detected by incubation with cy5 conjugated Alexa Fluor® 647 Goat Anti-Human IgG (H+L) (Invitrogen, Lofer, Austria), diluted in 1:10,000 in PBSTritonX 0.1% and 3% skim milk powder using rotating conditions for 1h, with a final washing step as outlined above. Microarrays were then scanned and fluorescent data extracted from images (Fig. 1) using the GenePixPro 6.0 software (AXON).

### Example 5: PSA testing

Prostate-specific antigen (PSA) is a 33-kDa glycoprotein with serine protease activity, found in large amounts in the prostate and seminal plasma. PSA measurement is widely accepted and the current diagnostic standard tool for prostatic cancer diagnostics (Stamey et al., 1987 N Engl J Med 1987;317:909 -15; Hudson et al., 1991 J Urol 1991;145:802-6).

The PSA ELISA test is based on the principle of a solid phase enzyme-linked immunosorbent assay. The assay system utilizes a PSA antibody directed against intact PSA for solid phase immobilization (on the microtiter wells). A monoclonal anti-PSA antibody conjugated to horseradish peroxidase (HRP) is in the antibody-enzyme conjugate solution. The test sample was allowed to react first with the immobilized rabbit antibody at room temperature for 60 minutes. The wells were washed to remove any unbound antigen. The monoclonal anti-PSA-HRP conjugate was then reacted with the immobilized antigen for 60 minutes at room temperature resulting in the PSA molecules being sandwiched between the solid phase and enzyme-linked antibodies.

The wells were washed to remove unbound-labeled antibodies. A solution of TMB Reagent was added and incubated at room temperature for 20 minutes, resulting in the development of a blue color. The color development was stopped with the addition of Stop Solution changing the color to yellow. The concentration of PSA is directly proportional to the color intensity of the test sample. Absorbance is measured spectrophotometrically. The results are reported as nanograms of PSA per milliliter (ng/mL) of blood. Sample signal data was calibrated with a set of standard concentrations.

### Example 6: Data analysis and permutation analysis

Data were 1) quantil normalised and alternatively 2) normalised with Combat transformation for removal of batch effects, when samples were processed on microarrays in 3 different runs; data analyses was conducted using BRB array tools (web at li-nus.nci.nih.gov/BRB-ArrayTools.html) upon quantile normalized data, and the R software upon the 2 different normalization strategies (quantil and Combat DWD normalized) followed by missing value imputation (Trevor Hastie, Robert Tibshirani, Bal-asubramanian Narasimhan and Gilbert Chu. impute: impute: Imputation for microarray data. R package version 1.42.0.).

For identification of tumour marker profiles and classifier markers, class prediction analyses applying cross-validation was used. Classifiers were built for distinguishing both classes of samples denoted "Carc" carcinoma patients, and "Contr" individuals with benign prostate hyperplasia.

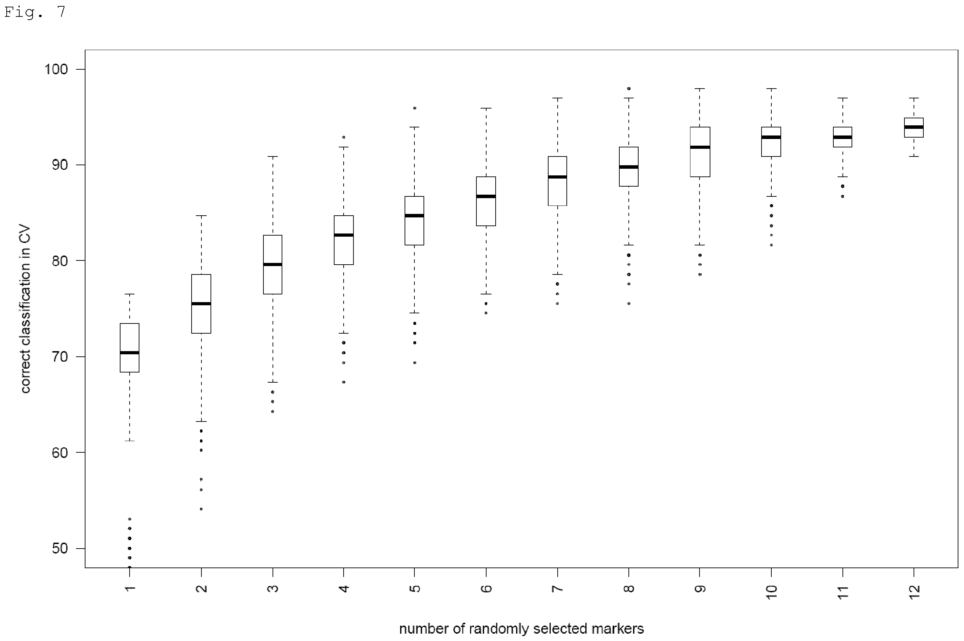

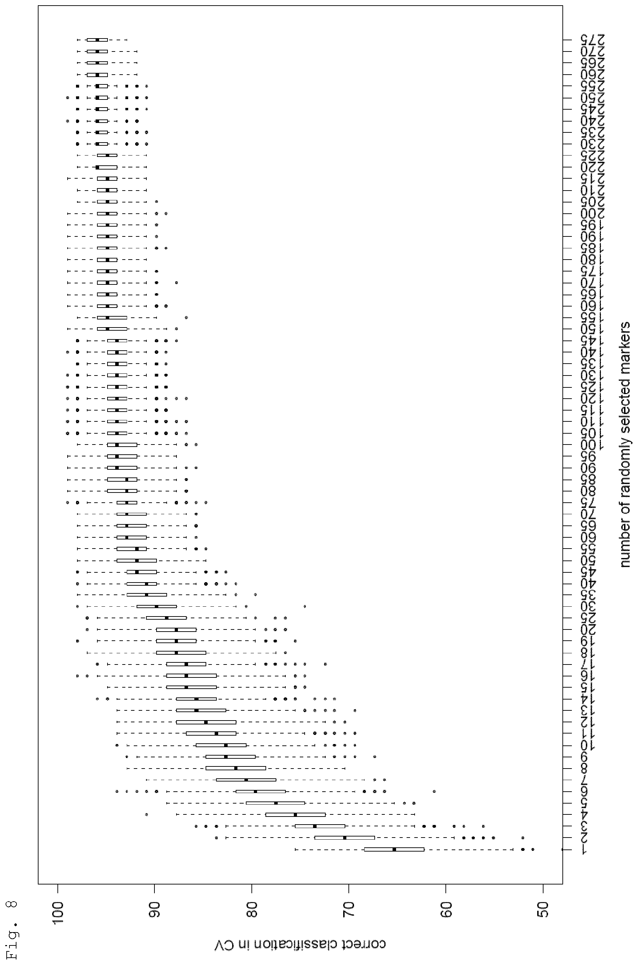

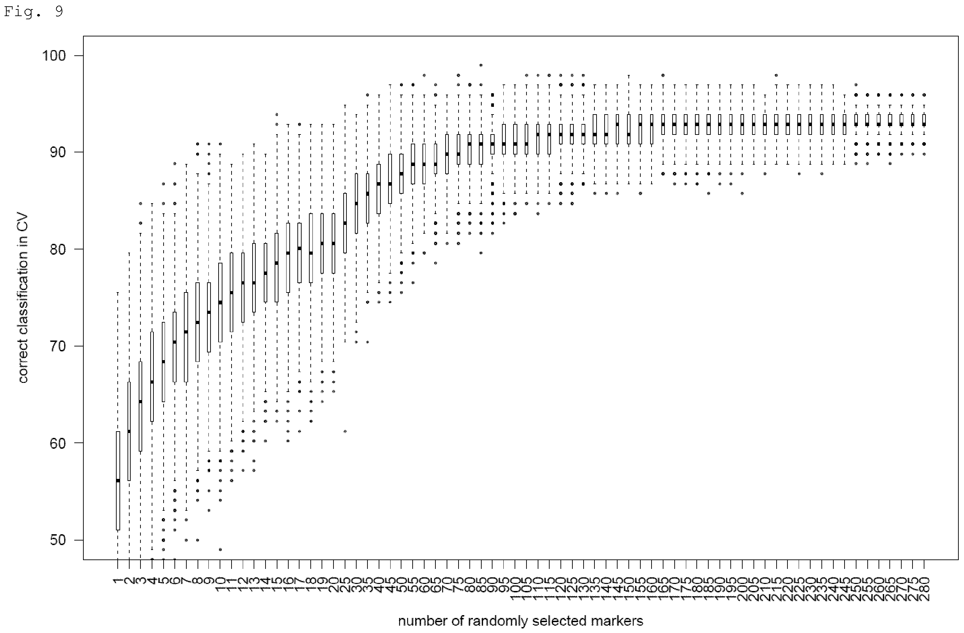

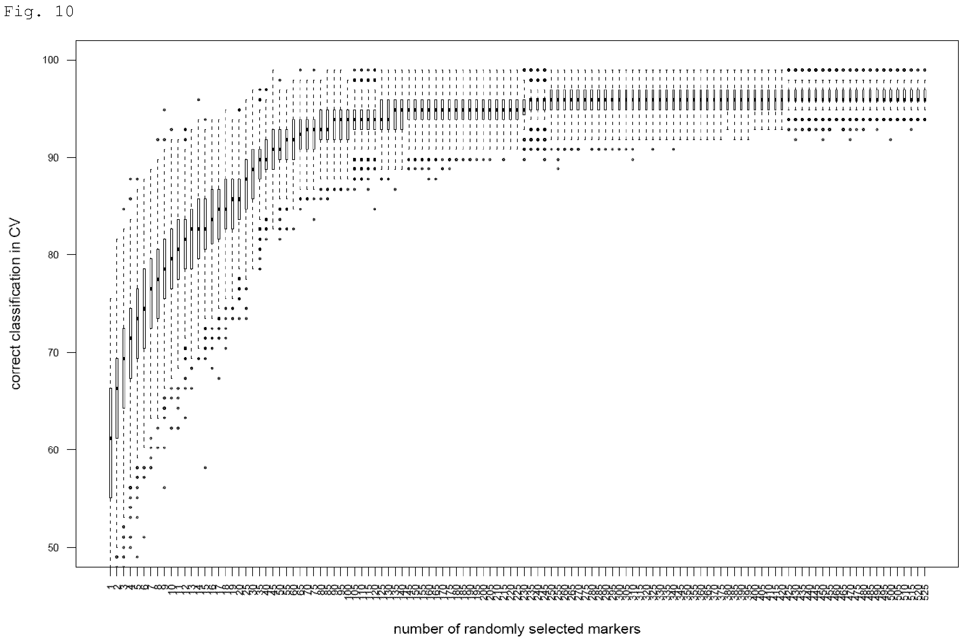

Due to the large redundancy of genes/proteins involved in biological processes (such as tumorigenesis), redundant lists of genes are covered, of which a subset can be used for classification. To show how many randomly chosen markers are necessary for the task of classifying tumor versus control, random sets of 1, 2, 3, ... markers are drawn from the marker lists and the classification accuracy in cross-validation is reported. Results are shown in Fig. 7-13.

### Example 7: Results Summary

For distinguishing 1) Controls vs Carcinomas, after different normalization strategies (quantil and Combat DWD normalized) followed by missing value imputation, the best 10 classifiers were chosen from claim 3, run 1. It was also shown that using only isolated or only 2 markers from the present classifier sets enables correct classification of 100% (Example 9.7). Therefore, the marker-lists, subsets and single markers (antigens; proteins; peptides) are of particular diagnostic values.

In addition, it has already been shown that peptides deduced from proteins or seroreactive antigens can be used for diagnostics and in the published setting even improve classification success (Syed 2012; Journal of Molecular Biochemistry; Vol 1, No 2, www.jmolbiochem.com/index.php/JmolBiochem/article/view/54).

### Example 8: Group results

Several lists of marker sets have been identified. All markers are grouped in List 4 recited above. Smaller marker selections portions are provided in Lists 2, 3, 3p1, 3p2 and 3p3. All markers are grouped together in List 4. Lists 3p1, 3p2 and 3p3 were pooled in list 3.

### List 2: 268 Marker proteins given by their gene symbol.

OXA1L, GOLM1, NRXN2, PAPSS1, GNAI2, FTSJD2, CERS1, FNTB, MYO19, ADCK3, DHCR24, TUBGCP2, LRFN5, PSA, ATAT1, SH3BGRL, LARP1, NPC2 (includes EG:10577), UNK, ATRX, PSMA7, LCMT1, VPS37D, MITD1, CRYGD, AKR1B1, PRKAR1B, ALKBH2, CCL2, GNAI2, MTF2 (includes EG:17765), RHOG, ARMCX1, LSM12 (includes EG:124801), WDR1, RSBN1L, LAMB2, DEDD2, NEUROD6, KRT8, STX6, MDFI, FBXW5, CYHR1, MGEA5, FAHD2B, EDC4, PSD, RPL36A, ZNF238, PIK3IP1, PPIA, PRKD2, DCP1A, LCAT, MYO1F, GSTM3, PRIC285, CRABP2, CCDC136, CSF1R, ARHGAP25, IDH2, NPM1, PAF1 (includes EG:361531), HNRPDL, COPZ1, PSMC3, PRDM8, ZNF514, UBR4, WDR73, RHOB, C19orf25, MMP14, LTBP3, NUP88, DPP9, SPSB3, TSKU, TNFAIP8L2, SYS1 (includes EG:336339), RPL37A, GSTM4, PKNOX1, DRAP1, HN1, BAG6, HSPA9, LRRC47, XRCC1 (includes EG:22594), CUX1, COPS6, NSUN5P1, PSAP, LSM14B, NCBP2, SDHA, FAM98C, MAD2L1, PPP2R1A, COL4A1, CYFIP1, PRDX5, FAM220A, RPS7, EZR, EXOSC8, FAM20C, SRA1, ETS2, SLA, SERPINA1, LARS, SLIT1, FHL1 (includes EG:14199), PTPRA, ELAVL3, BBIP1, HNRNPH1, PLXNA1, PPP2R1A, IVNS1ABP, PRDX1, THOC3, PELI1, PHF2, OCIAD2, PAK6, FIS1 (includes EG:288584), IL16, IDH1, SRSF1, PABPC1, C8orf33, ARHGEF18, ACTR1B, ANKS3, ZC3H12A, PCBP1, SRM, STMN4, EPC1, NLRP1, PTOV1, C12orf51, WDR1, TCF19, ZXDC, VARS, HTATIP2, PCM1, ATCAY, PRDX3, NSD1, DUS1L, GABARAP, FAM21A/FAM21C, SPRY1, ADAR, KNDC1, HMGN2, AHCTF1, NFKB1, DCHS1, CARHSP1, CORO7/CORO7-PAM16, SSR4, KIAA1109, ABT1, PCDH7, AXIN1, TPX2, SH2B1, RPS4Y1, AKR1C4, PAM, UNC13B, HLA-C, NUDT16L1, ZNF462, NPC2 (includes EG:10577), PUM1, EDF1, COMT, PSMB10, LSM14B, SNF8, CTSW, MTUS1, ARID5A, PSMC4, KIAA0753, EPS15L1, ABHD8, HK1, DNM2, WASL, VPS18, ASF1B, VAV2, PPAP2B, HDAC2, SNRPD3, MICU1, C1orf131, NTAN1, SCG5, REC8 (includes EG:290227), LRPPRC, PPOX, ENOl, PCDHB14, PLA2G2A, THOC3, PAFAH1B3, PTK7, SERBP1, HNRNPA1, RASGRP2, NUP88, FAM118B, TNKS1BP1, H19, NECAP2, PLBD1, CFL1, ITGA3, ZNF668, CDKN2D, RHOT2, AKT2, NARFL, PPP2R3B, ABTB1, EMILIN1, TBC1D9B, PKM, ADNP, PPP1R12A, MRC2, PPIL1, TNKS1BP1, FGB, PPIE, SRSF4, BLOC1S1, CNPY3, IRF3, WRB, TOP2B, PDXDC1, TCERG1, CAPZB, BABAM1, HSPA5, CNOT3, EIF3C/EIF3CL, IL17RA, OGFR, BIRC2, LCP2, GSTP1, MYH9 (includes EG:17886), PIK3R5, NCKAP5L, RGS1, MAN2C1, EHD1, USP5, PLEC, SLC35A2, RPL10A, ARHGDIA, COPE, KDM3A, SMARCC2

### List 3: 282 Marker proteins given by their gene symbol.

NRXN2, CERS1, MYO19, LRFN5, ATAT1, KRT8, FBXW5, MGEA5, RPL36A, PRKD2, DCP1A, MYO1F, ARHGAP25, HNRPDL, COPZ1, UBR4, WDR73, SPSB3, LRRC47, NSUN5P1, MAD2L1, SLA, FHL1 (includes EG:14199), IDH1, IL16, SRSF1, ZC3H12A, ACTR1B, LCK, VARS, SPRY1, SSR4, TPX2, RPS4Y1, ARID5A, PSMC4, SFTPB, WASL, RASGRP2, TK1, RHOT2, PPP2R3B, ABTB1, PPIL1, IRF3, CRAT, EIF3C/EIF3CL, DUT, GIPC1, LMTK2, CDC37, LCP2, FOSB, ARFRP1, GSTP1, MTCH1, PSMB5, HIST3H2A, PIK3R5, C9orf86, DDX39B, TINAGL1, INPPL1, MAN2C1, PRKCZ, DDOST, USP5, PLEC, HARS, RPL10A, C22orf46, KRBA1, NFATC3, ATP5D, SMYD4, E2F1, PIK3R2, CLIC1, USP28, MORF4L1, POLR2G, TRIM78P, COG4, RHOT2, TACC2, YWHAE, IP6K2, IKBKB, AKR1B1, CACNA1E, POTEE/POTEF, KLHL23/PHOSPHO2-KLHL23, MEPCE, EIF5A, DOCK9, PLXNB2, NR4A1, RPL4, MBD1, VCP, H19, RARA, CDH2, KIF2A, FXYD5, PPA1, EEF1G, RIC8A, ZNF12, B4GALT2, FNDC4, CYR61, OBFC1, WASH1/WASH5P, HSPA4, PBXIP1, WASH1/WASH5P, PLCG1, HMGB2, GTF2F1, UBC, CELF3, KIF1A, KARS, RNF216, TGS1, NFIX, SGSH, PLEKHO1, TAOK2, MLL5, LAMB1, ZNF431, C17orf28, BAZ1B, UHRF2, ATP5SL, PEX7, TSC2, TMSB10/TMSB4X, LIMS2, TBC1D13, UROD, KLF4, BZW2, SULF2, HLA-E, PRRC2A, TBC1D2, H3F3A/H3F3B, GRK6, HIP1R, ARPC5L, NFKB2, SF3B2, PSMC3, ARPC1B, MGA, C1orf122, SYNE2, NOA1, INPP5F, CDK5RAP3, PABPC1, MDN1, LARP4B, UBE3C, HAGH, NIN, HDAC10, RPS4Y2, GMIP, CCDC88C, ATP1B3, SPOCK2, CYFIP2, TAF1C, WDR25, BAZ1A, NFKBIA, HLA-B, TYK2, C19orf6, SERBP1, SLC25A3, QARS, PPP1R9B, DOCK2, AP2S1, DIS3L, CCNB1IP1, ZNF761, MKS1 (includes EG:287612), FCHO1, TYMP, COQ6, TELO2, XPNPEP3, TXNDC11, HIVEP3, CD44, KPNB1, PCBP2, NPEPL1, PLCB2, FBXO6, PRMT1, ATXN7L2, TADA3, MRPL38 (includes EG:303685), PTBP1, MAGED4/MAGED4B, SEC16A, SLC35B2, ADAMTS10, ZNF256, GBAS, DNMT3A, KCNJ14, PEPD, PITRM1, LSM14A, NDUFV1, TOX2, CAD, HCFC1, WDR11, POLR2J4, TOLLIP, CHGA, HDAC1, HSP90AB1, KLF5, UQCRC1, GALK1, KIAA1731, HSPG2, TLN1, TMED3, DUS2L, LOC407835, TNRC6B, PKM, DAK, VDAC1, LRP4, ULK3, PHKB, NBEA, GTF3C1, IVNS1ABP, AHCY, WDR82, HACL1, USP22, KIF2A, APO-BEC3A, TTC27, YWHAQ, SEC24B, ZNF439, HTRA1, WDTC1, LARP7, BIN3, PTPRO, GET4, SUPV3L1, DHX34, PDZD4, MYCBP2, GATA1, USP39, DFFA, USP7, ATP8B3, UBE2N, C17orf28, EIF3C/EIF3CL, IMPDH1, SART3, ANXA1.

Each of these markers has a high correct classification accuracy if taken alone. Classification accuracy is given in the following table by their AUC (area-under-curve) classification values:

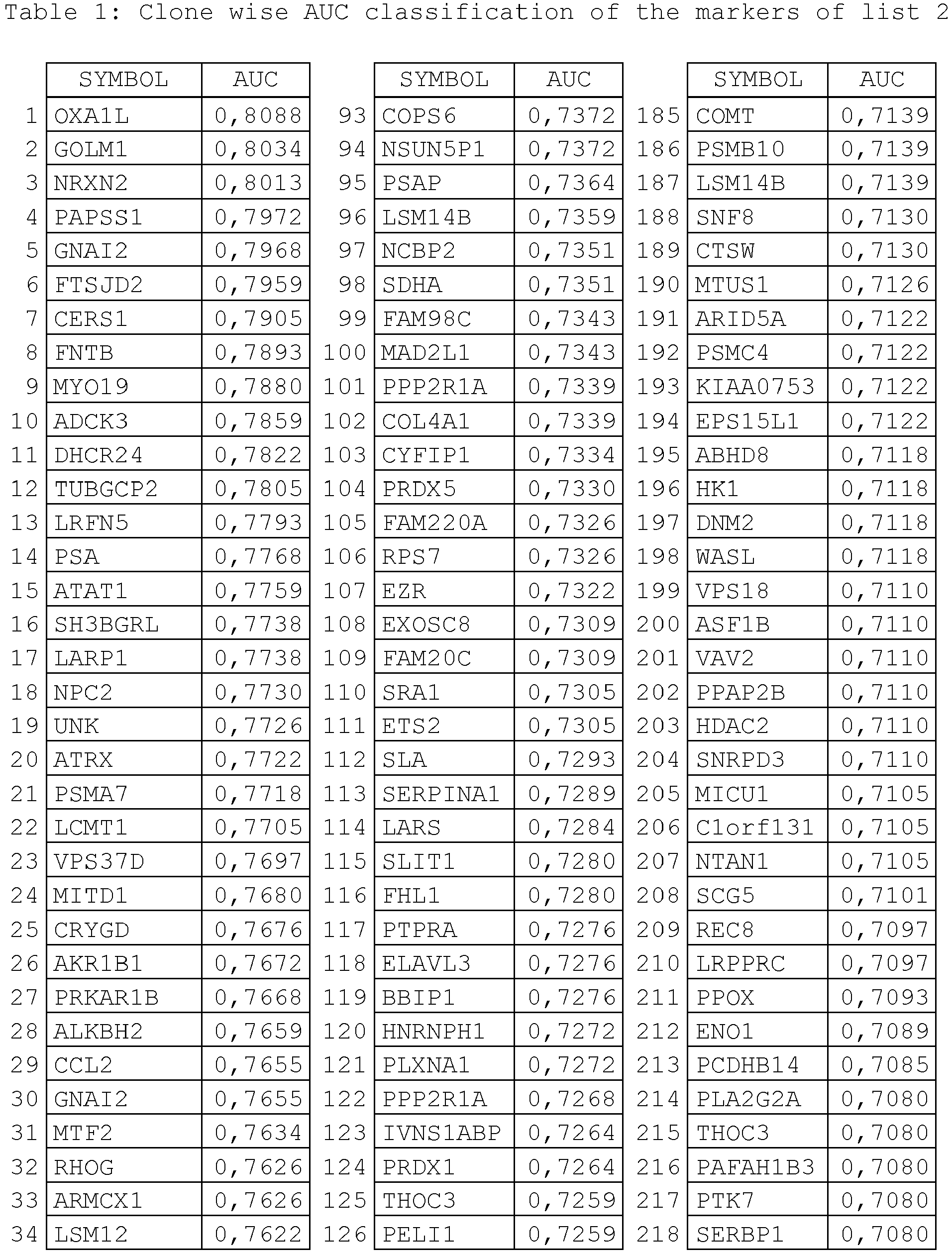

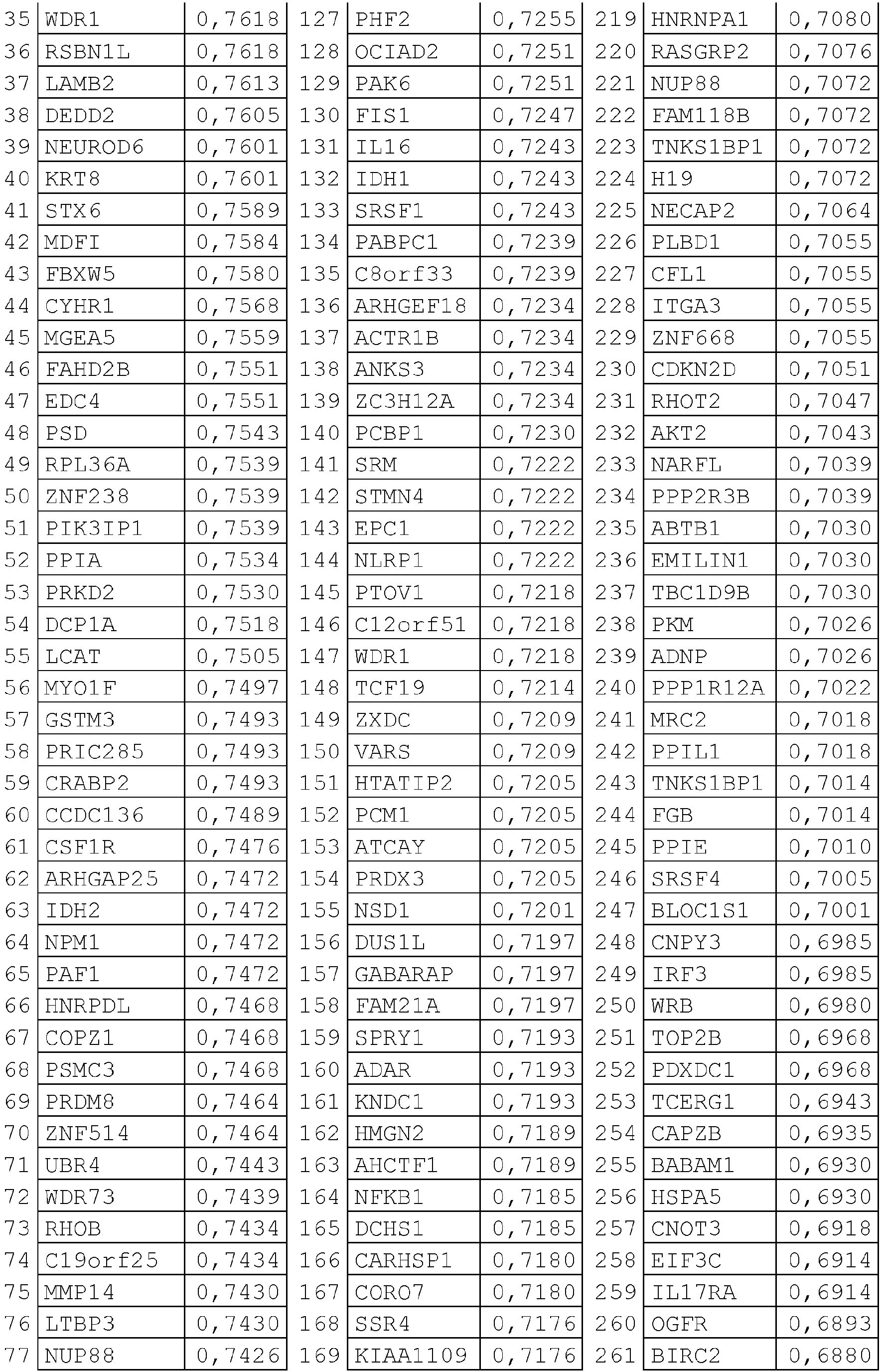

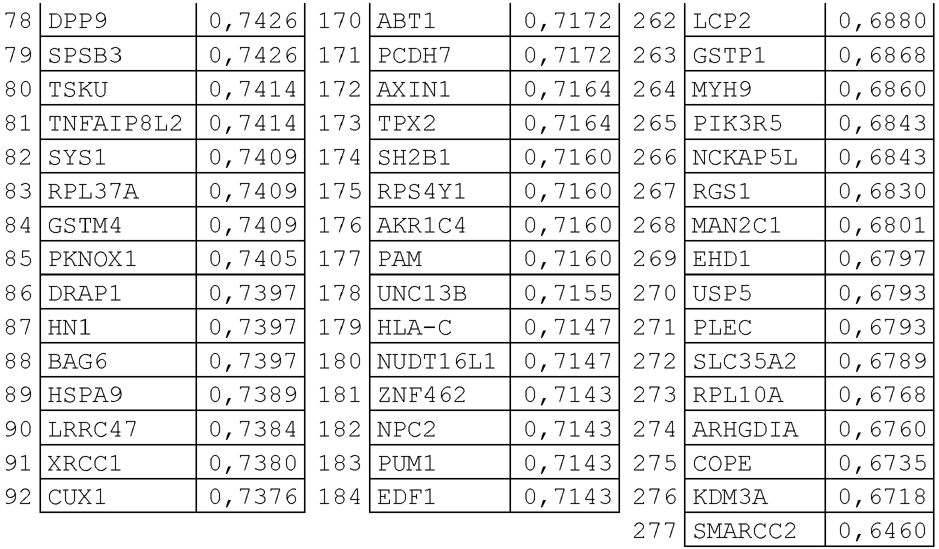

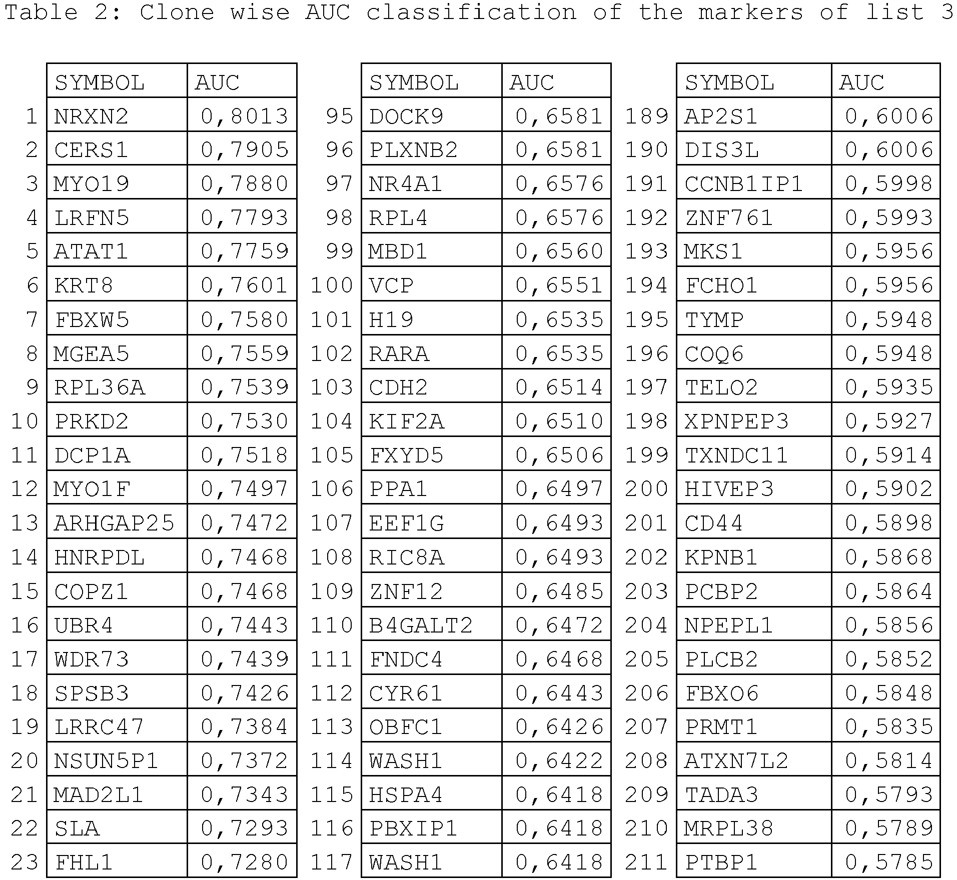

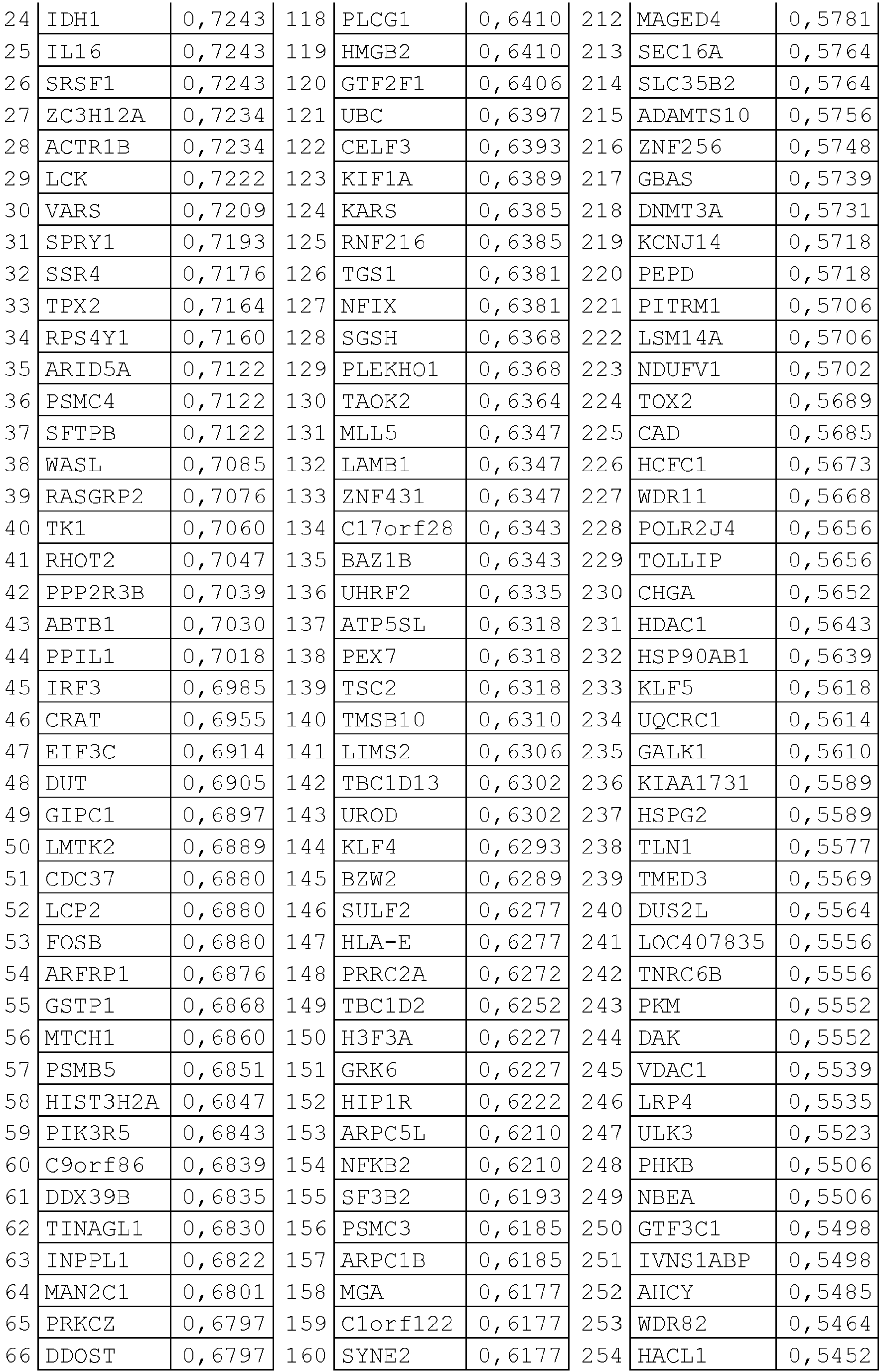

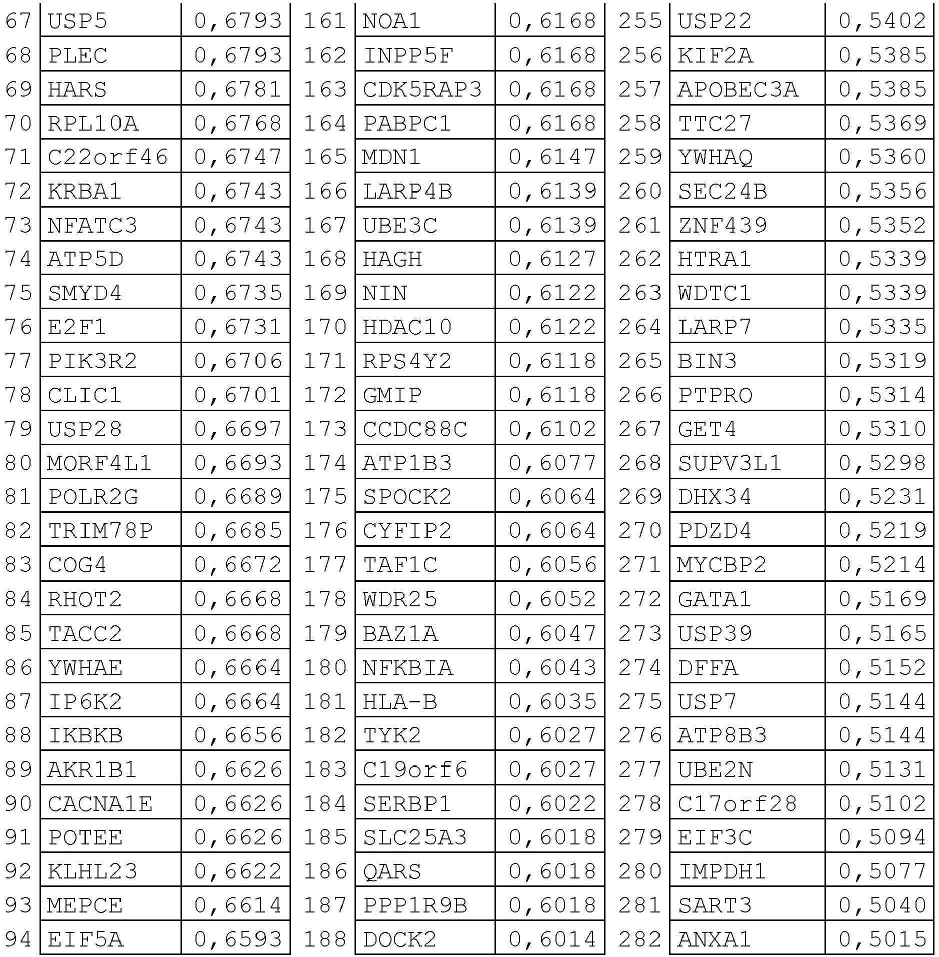

These markers are especially potent when used in combination with other markers. Figures 7-10 show a random permutation analysis of these markers when taken alone or in any combination of 2, 3, 4 or more markers.

When splitting the markers of list 3 into the following subgroups, even higher correct classification results from low numbers of random markers of these lists were obtained (see Fig. 11-13). The subgroups are:

### List 3p1:

NRXN2, LRFN5, KRT8, FBXW5, MGEA5, DCP1A, MYO1F, ARHGAP25, WDR73, NSUN5P1, FHL1 (includes EG:14199), IDH1, VARS, SPRY1, PSMC4, SFTPB, WASL, RASGRP2, TK1, RHOT2, PPP2R3B, PPIL1, GIPC1, LMTK2, CDC37, FOSB, PIK3R5, C22orf46, NFATC3, E2F1, MORF4L1, YWHAE, CACNA1E, RPL4, VCP, RARA, KIF2A, EEF1G, B4GALT2, PBXIP1, GTF2F1, RNF216, TGS1, NFIX, TAOK2, MLL5, ZNF431, TMSB10/TMSB4X, LIMS2, PRRC2A, TBC1D2, GRK6, PSMC3, MGA, C1orf122, MDN1, LARP4B, NIN, CCDC88C, SPOCK2, NFKBIA, C19orf6, DOCK2, AP2S1, COQ6, TXNDC11, HIVEP3, PLCB2, PTBP1, DNMT3A, KCNJ14, LSM14A, CHGA, KLF5, GALK1, DUS2L, NBEA, WDR82, USP22, KIF2A, BIN3, PTPRO, USP39, UBE2N, ANXA1.

### List 3p2:

NRXN2, MYO19, ATAT1, RPL36A, UBR4, SPSB3, LRRC47, IL16, ZC3H12A, LCK, TPX2, RPS4Y1, ABTB1, IRF3, EIF3C/EIF3CL, DUT, LCP2, ARFRP1, GSTP1, DDX39B, MAN2C1, PRKCZ, USP5, PLEC, HARS, RPL10A, KRBA1, CLIC1, USP28, POLR2G, TRIM78P, RHOT2, TACC2, IP6K2, IKBKB, EIF5A, NR4A1, MBD1, CDH2, FXYD5, RIC8A, FNDC4, OBFC1, HMGB2, UBC, SGSH, LAMB1, UHRF2, PEX7, TSC2, TBC1D13, SULF2, HLA-E, HIP1R, NFKB2, SF3B2, ARPC1B, SYNE2, CDK5RAP3, CYFIP2, BAZ1A, HLA-B, TYK2, SERBP1, DIS3L, ZNF761, TYMP, XPNPEP3, CD44, SEC16A, PEPD, HCFC1, HSP90AB1, UQCRC1, TLN1, DAK, PHKB, GTF3C1, HTRA1, DFFA, ATP8B3, UBE2N.

### List 3p3:

CERS1, KRT8, PRKD2, HNRPDL, COPZ1, MAD2L1, SLA, SRSF1, ACTR1B, SSR4, ARID5A, CRAT, MTCH1, PSMB5, HIST3H2A, C9orf86, TINAGL1, INPPL1, DDOST, ATP5D, SMYD4, PIK3R2, COG4, AKR1B1, POTEE/POTEF, KLHL23/PHOSPHO2-KLHL23, MEPCE, DOCK9, PLXNB2, H19, PPA1, ZNF12, CYR61, WASH1/WASH5P, HSPA4, WASH1/WASH5P, PLCG1, CELF3, KIF1A, KARS, PLEKHO1, C17orf28, BAZ1B, ATP5SL, UROD, KLF4, BZW2, H3F3A/H3F3B, ARPC5L, NOA1, INPP5F, PABPC1, UBE3C, HAGH, HDAC10, RPS4Y2, GMIP, ATP1B3, TAF1C, WDR25, SLC25A3, QARS, PPP1R9B, CCNB1IP1, MKS1 (includes EG:287612), FCHO1, TELO2, KPNB1, PCBP2, NPEPL1, FBXO6, PRMT1, ATXN7L2, TADA3, MRPL38 (includes EG:303685), MAGED4/MAGED4B, SLC35B2, ADAMTS10, ZNF256, GBAS, PITRM1, NDUFV1, TOX2, CAD, WDR11, POLR2J4, TOLLIP, HDAC1, KI-AA1731, HSPG2, TMED3, LOC407835, TNRC6B, PKM, VDAC1, LRP4, ULK3, IVNS1ABP, AHCY, HACL1, APOBEC3A, TTC27, YWHAQ, SEC24B, ZNF439, WDTC1, LARP7, GET4, SUPV3L1, DHX34, PDZD4, MYCBP2, GATA1, USP39, USP7, C17orf28, EIF3C/EIF3CL, IMPDH1, SART3.

### Example 9: Detailed Results

### Example 9.1: "Carc vs. Contr" - Top 10 genes selected by their AUC value.

The following markers were identified according to this example *(Quantil-normalised data):*

### List 1: 12 Marker proteins given by their gene symbol:

OXA1L, GOLM1, NRXN2, PAPSS1, GNAI2, FTSJD2, CERS1, FNTB, MYO19, ADCK3, SDHA, FAM184A

| SYMBOL | AUC |
|---|---|
| OXA1L | 0.80883 |
| GOLM1 | 0.803415 |
| NRXN2 | 0.801333 |
| PAPSS1 | 0.797168 |
| GNAI2 | 0.796751 |
| FTSJD2 | 0.795918 |
| CERS1 | 0.790504 |
| FNTB | 0.789254 |
| MYO19 | 0.788005 |
| ADCK3 | 0.785923 |
| SDHA | 0.73511 |
| FAM184A | 0.556018 |

### Example 9.2: "Carc vs Contr" - 8 greedy pairs algorithm- >1NN 100%

The following markers were identified according to this example *(Quantil-normalised data):*

### List 5: 16 Marker proteins given by their gene symbol:

ATAT1, CCDC136, CDK5RAP3, GOLGA4, HCFC1, HLA-C, HNRNPA1, MYO19, NONO, PLEC, PPP1R9B, SNX9, SULF2, USP5, WDR1 and ZC3H12A.

The "greedy pairs" strategy was used for class prediction of the first 36 (18 carcinoma; 18 control) samples of run2, and it was possible to very efficiently build a classifier for distinguishing "Carc" versus "Contr". Using "8 greedy pairs" of features on arrays, the 1-Nearest Neighbour Predictor (1-NN) enabled correct classification of 100% of samples.

Greedy pairs algorithm was used to select 8 pairs of genes. Repeated 1 times K-fold (K= 20) cross-validation method was used to compute misclassification rate.

**Performance of classifiers during cross-validation.**

| | **Compound Covariate Predictor Correct?** | **Diagonal Linear Discriminant Analysis Correct?** | **1-Nearest Neigh bor** | **3-Nearest Neigh bors Correct?** | **Nearest Centro id Correct?** | **Support Vector Machines Correct?** | **Bayesian Compound Covariate Predictor Correct?** |
|---|---|---|---|---|---|---|---|
| **Mean percent of correct classification:** | 92 | 94 | 100 | 94 | 92 | 94 | 94 |

**Performance of the 1-Nearest Neighbor Classifier:**

| **Class** | **Sensitivity** | **Specificity** | **PPV** | **NPV** |
|---|---|---|---|---|
| **Case** | 1 | 1 | 1 | 1 |
| **Control** | 1 | 1 | 1 | 1 |

### Example 9.3: "Carc vs. Contr" - p<5e-06 → 100%

The following markers were identified according to this example *(Quantil-normalised data):*

### List 6: 13 Marker proteins given by their gene symbol:

ARID5A, EIF3C, FCHO1, HAGH, IVNS1ABP, KLHL23, LARP7, NDUFS2, PLXNB2, SMARCC2, TOLLIP, TRIO and WDR11.

Genes significantly different between the classes at 5e-06 significance level were used for class prediction for the first 28 (14 carcinoma; 14 control) samples of run3, and it was possible to very efficiently build classifiers for distinguishing "Contr" versus "Carc". The Diagonal Linear Discriminant Analysis (DLDA) and 3-Nearest Neighbor Predictor (3-NN) enabled best correct classification of 100% of samples.

Genes significantly different between the classes at 5e-06 significance level were used to select genes. Leave-one-out cross-validation method was used to compute misclassification rate.

**Performance of classifiers during cross-validation.**

| | **Compound Covariate Predictor Correct?** | **Diagonal Linear Discriminant Analysis Correct?** | **1-Nearest Neig hbor** | **3-Nearest Neigh bors Correct?** | **Near-rest Centro id Correct?** | **Support Vector Machines Correct?** | **Bayesian Compound Covariate Predictor Correct?** |
|---|---|---|---|---|---|---|---|
| **Mean percent of correct classification:** | 96 | 100 | 96 | 100 | 96 | 93 | 96 |

**Performance of the Diagonal Linear Discriminant Analysis Classifier:**

| **Class** | **Sensitivity** | **Specificity** | **PPV** | **NPV** |
|---|---|---|---|---|
| **Case** | 1 | 1 | 1 | 1 |
| **Control** | 1 | 1 | 1 | 1 |

**Performance of the 3-Nearest Neighbors Classifier:**

| **Class** | **Sensitivity** | **Specificity** | **PPV** | **NPV** |
|---|---|---|---|---|
| **Case** | 1 | 1 | 1 | 1 |
| **Control** | 1 | 1 | 1 | 1 |

### Example 9.4: "Carc vs. Contr" - p<0.000005 →91%

The following markers were identified according to this example *(Quantil-normalised data):*

### List 7: 17 Marker proteins given by their gene symbol:

AKR1C4, B4GALT2, BRD9, COPS6, EEFSEC, HCFC1, MYO1F, NBEA, NEUROD2, PPP1CA, PSMC4, RASGRP2, RPA3, SMG8, SUGP1, TMEM131 and TUBB2B.

As in the previous example, genes significantly different between the classes at 5e-06 significance level were used for class prediction for the first 35 (18 carcinoma; 17 control) samples of run 1, and it was possible to very efficiently build classifiers for distinguishing "Carc" versus "Contr". The 1-Nearest Neighbor Predictor (1-NN) enabled best correct classification of 91% of samples.

Genes significantly different between the classes at 5e-06 significance level were used to select genes. Leave-one-out cross-validation method was used to compute misclassification rate.

**Performance of classifiers during cross-validation.**

| | **Compound Covariate Predictor Correct?** | **Diagonal Linear Discriminant Analysis Correct?** | **1-Nearest Neigh bor** | **3-Nearest Neigh bors Correct?** | **Nearest Centro id Correct?** | **Support Vector Machines Correct?** | **Bayesian Compound Covariate Predictor Correct?** |
|---|---|---|---|---|---|---|---|
| **Mean percent of correct classification:** | **89** | **86** | **91** | **89** | **89** | **86** | **90** |

**Performance of the 1-Nearest Neighbor Classifier:**

| **Class** | **Sensitivity** | **Specificity** | **PPV** | **NPV** |
|---|---|---|---|---|
| **Case** | 1 | 0.824 | 0.857 | 1 |
| **Control** | 0.824 | 1 | | 0.857 |

### Example 9.5: "Carc vs. Contr" - Best discriminatory power

The top ten genes (by AUC value) discriminating between the classes from claim 1 were used for search of the best discriminatory power. A best subset selection was created by starting with the best discriminator (by cross-validated prediction accuracy using SVM) and sequentially adding new features from claim 1 which most improve classification accuracy. This was repeated for the first 10 features.

| SYMBOL | CV accuracy |
|---|---|
| NRXN2 | 74.31973 |
| GNAI2 | 80.13605 |
| PAPSS1 | 86.90476 |
| CERS1 | 89.52381 |
| GOLM1 | 93.60544 |
| MYO19 | 93.91156 |
| ADCK3 | 95.81633 |
| FAM184A | 95.57823 |
| FNTB | 95.57823 |
| SDHA | 94.79592 |

### List 8: 10 Marker proteins given by their gene symbol:

NRXN2, GNAI2, PAPSS1, CERS1, GOLM1, MYO19, ADCK3, FAM184A, FNTB, SDHA (see Fig. 1 for accuracy of best subset selection)

### Example 9.6: "Carc vs. Contr" - Best discriminatory power

The top ten genes (by AUC value) discriminating between the classes from claim 2 were used for search of the best discriminatory power. A best subset selection was created by starting with the best discriminator (by cross-validated prediction accuracy using SVM) and sequentially adding new features from claim 2 which most improve classification accuracy. The following is the list of the best subset selection. This was repeated for the first 20 features.

| Symbol | CV accuracy (SVM) |
|---|---|
| PSMA7 | 74.38776 |
| PSA | 83.60544 |
| NRXN2 | 89.82993 |
| PAPSS1 | 94.4898 |
| FAM20C | 95.47619 |
| NUP88 | 98.26531 |
| PTOV1 | 99.69388 |
| DRAP1 | 99.96599 |
| ASF1B | 99.96599 |
| CAPZB | 100 |
| PCBP1 | 100 |
| PPP1R12A | 100 |
| PSMC4 | 100 |
| LTBP3 | 100 |
| FNTB | 99.96599 |
| EDC4 | 99.7619 |
| SSR4 | 99.72789 |
| SMARCC2 | 99.79592 |
| LAMB2 | 99.96599 |

### List 9: 19 Marker proteins given by their gene symbol:

PSMA7, PSA, NRXN2, PAPSS1, FAM20C, NUP88, PTOV1, DRAP1, ASF1B, CAPZB, PCBP1, PPP1R12A, PSMC4, LTBP3, FNTB, EDC4, SSR4, SMARCC2, LAMB2, GOLM1 (see Fig. 2 for accuracy of best subset selection)

### Example 9.7: "Carc vs. Contr" - Best discriminatory power

Genes significantly different between the classes from claim 3, run 1 were used for search of the best discriminatory power. The following is the list of the best subset selection.

| Symbol | CV accuracy (SVM) |
|---|---|
| PSMC4 | 93.33333 |
| DNMT3A | 100 |
| TGS1 | 100 |
| NRXN2 | 100 |
| GRK6 | 100 |
| TBC1D2 | 100 |
| ZNF431 | 100 |
| DUS2L | 100 |
| MGA | 100 |

### List 10: 9 Marker proteins given by their gene symbol.

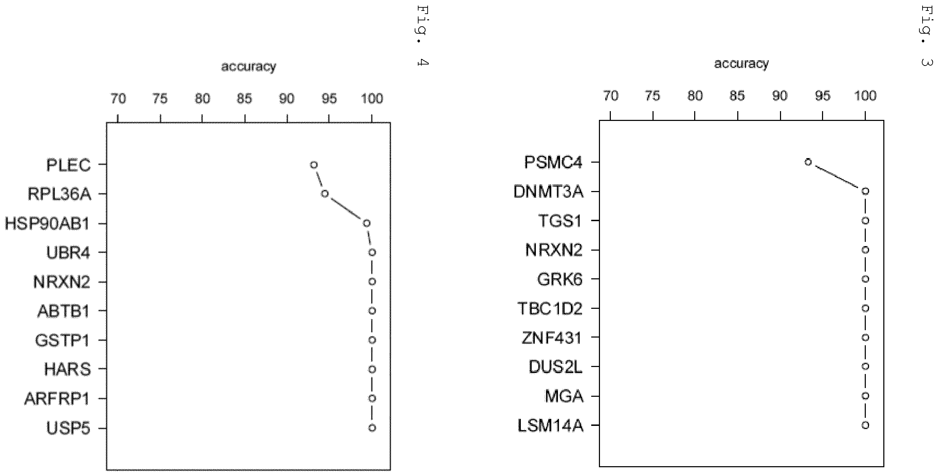

PSMC4, DNMT3A, TGS1, NRXN2, GRK6, TBC1D2, ZNF431, DUS2L, MGA, LSM14A (see Fig. 3 for accuracy of best subset selection)

### Example 9.8: "Carc vs. Contr" - Best discriminatory power

Genes significantly different between the classes from claim 3, run 2 were used for search of the best discriminatory power. The following is the list of the best subset selection.

| Symbol | CV accuracy (SVM) |
|---|---|
| PLEC | 93.2381 |
| RPL36A | 94.47619 |
| HSP90AB1 | 99.42857 |
| UBR4 | 100 |
| NRXN2 | 100 |
| ABTB1 | 100 |
| GSTP1 | 100 |
| HARS | 100 |
| ARFRP1 | 100 |
| USP5 | 100 |

### List 11: 10 Marker proteins given by their gene symbol:

PLEC, RPL36A, HSP90AB1, UBR4, NRXN2, ABTB1, GSTP1, HARS, ARFRP1, USP5 (see Fig. 4 for accuracy of best subset selection)

### Example 9.9: "Carc vs. Contr" - Best discriminatory power

Genes significantly different between the classes from claim 3, run 3 were used for search of the best discriminatory power. The following is the list of the best subset selection.

| Symbol | CV accuracy (SVM) |
|---|---|
| HIST3H2A | 97.02381 |
| RPS4Y2 | 100 |
| HAGH | 100 |
| HNRPDL | 100 |
| COPZ1 | 100 |
| CRAT | 100 |
| GET4 | 100 |
| SUPV3L1 | 100 |
| ACTR1B | 100 |
| UBE3C | 100 |

### List 12: 10 Marker proteins given by their gene symbol:

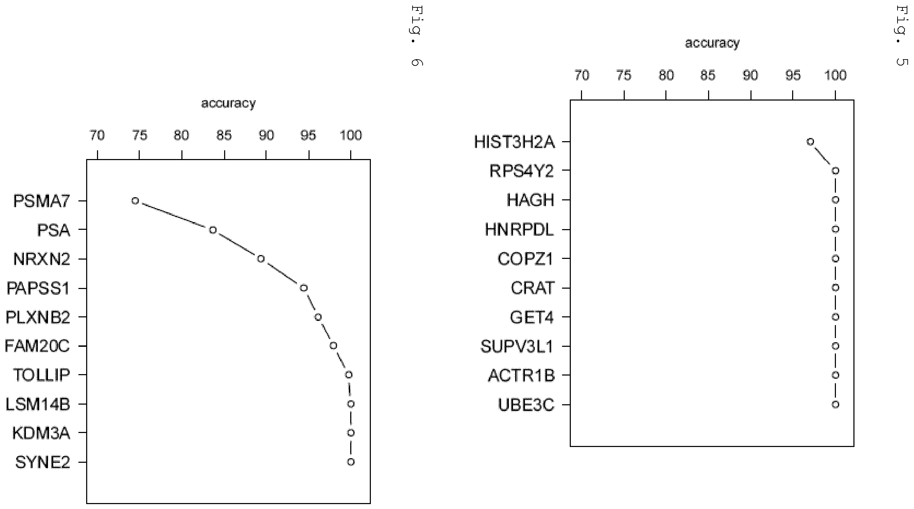

HIST3H2A, RPS4Y2, HAGH, HNRPDL, COPZ1, CRAT, GET4, SUPV3L1, ACTR1B, UBE3C (see Fig. 5 for accuracy of best subset selection)

### Example 9.10: "Carc vs. Contr" - Best discriminatory power

Genes significantly different between the classes from claim 4 were used for search of the best discriminatory power. The following is the list of the best subset selection.

| Symbol | CV accuracy (SVM) |
|---|---|
| PSMA7 | 74.42177 |
| PSA | 83.60544 |
| NRXN2 | 89.42177 |
| PAPSS1 | 94.42177 |
| PLXNB2 | 96.15646 |
| FAM20C | 97.92517 |
| TOLLIP | 99.69388 |
| LSM14B | 99.96599 |
| KDM3A | 100 |
| SYNE2 | 99.96599 |

### List 13: 10 Marker proteins given by their gene symbol:

PSMA7, PSA, NRXN2, PAPSS1, PLXNB2, FAM20C, TOLLIP, LSM14B, KDM3A, SYNE2 (see Fig. 6 for accuracy of best subset selection).

## Claims

1. Method of diagnosing prostate cancer or the risk of prostate cancer in a patient by detecting antibodies against the following marker proteins or a selection of at least 2 of the marker proteins or at least 20 % of the marker proteins selected from OXA1L, GOLM1, NRXN2, PAPSS1, GNAI2, FTSJD2, CERS1, FNTB, MYO19, ADCK3, SDHA, FAM184A (List 1) in a patient, wherein the selection comprises at least OXA1L, comprising the step of detecting antibodies binding said marker proteins in a sample of the patient.

2. Method of diagnosing prostate cancer or the risk of prostate cancer in a patient by detecting antibodies against at least 2 of the marker proteins or at least 20 % of the marker proteins selected from the markers of any one of List 2, 3, 4 or any combination thereof in a patient, wherein the markers comprise at least OXA1L, comprising the step of detecting antibodies binding said marker proteins in a sample of the patient.

3. Method according to claim 2 comprising detecting an antibody against a marker protein selected from any one of Lists 5, 6, 7, 8, 9, 10, 11, 12 or 13 in a patient, comprising the step of detecting antibodies binding said marker protein in a sample of the patient.

4. Method according to claim 2 comprising detecting antibodies against at least 2 or at least 20 % of the marker proteins selected from the markers of any one of Lists 5, 6, 7, 8, 9, 10, 11, 12 or 13 in a patient, comprising the step of detecting antibodies binding said marker proteins in a sample of the patient.

5. Method according to claim 2 comprising detecting antibodies against at least 2 or at least 20 % of the marker proteins selected from the markers of any one of Lists 3p1, 3p2, 3p3 in a patient, comprising the step of detecting antibodies binding said marker proteins in a sample of the patient.

6. Method according to any one of claims 1 to 5, comprising detecting at least markers SDHA and/or FAM184A in a patient, comprising the step of detecting antibodies binding said marker proteins in a sample of the patient.

7. Method according to any one of claims 1 to 6, further comprising detecting PSA in a sample from a patient comprising the step of detecting said marker protein or antigenic fragments thereof in a sample of the patient.

8. Method according to claim 7, wherein PSA protein in the sample is detected by an affinity assay, preferably with an immobilized affinity capturing agent.

9. The method of any one of claims 1 to 8, wherein the step of detecting antibodies binding said marker proteins comprises comparing said detection signal with detection signals of a healthy control and comparing said detection signals, wherein an increase in the detection signal indicates prostate cancer.

10. The method of any one of claims 1 to 9, a) wherein the step of detecting antibodies binding said marker proteins comprises comparing said detection signal with detection signals of one or more known prostate cancer control sample, preferably wherein the control signals are used to obtain a marker dependent signal pattern as indication classifier and the marker dependent signals of the patient are compared with and/or fitted onto said pattern, thereby obtaining information of the diagnosed condition.

11. The method of any one of claims 1 to 10, a) wherein the step of detecting antibodies binding said marker proteins comprises comparing said detection signal with detection signals of a cancerous control and comparing said detection signals, wherein a detection signal from the sample of the patient in amplitude of at least 60%, preferably at least 80%, of the cancerous control indicates prostate cancer; or b) wherein a detection signal in at least 60%, preferably at least 75%, of the used markers indicates prostate cancer.

12. A kit of diagnostic agents suitable to detect antibodies against any marker or marker combination as defined in claims 1 to 9, wherein the marker or marker combination comprises at least OXA1L, wherein said diagnostic agents comprise marker proteins or antigenic fragments thereof suitable to bind antibodies in a sample, the kit further comprising a computer-readable medium or a computer program product, comprising signal data for control samples with known conditions selected from prostate cancer, and/or calibration or training data for analysing said markers provided in the kit for diagnosing prostate cancer or distinguishing conditions selected from healthy conditions and prostate cancer.

13. The kit of claim 12 wherein said diagnostic agents are immobilized on a solid support.

14. The kit of claim 12 or 13 comprising a labelled secondary antibody, preferably for detecting an Fc part of antibodies of the patient.

15. The kit of claim 12, 13 or 14 comprising at most 3000 diagnostic agents, preferably at most 2500 diagnostic agents, at most 2000 diagnostic agents, at most 1500 diagnostic agents, at most 1200 diagnostic agents, at most 1000 diagnostic agents, at most 800 diagnostic agents, at most 500 diagnostic agents, at most 300 diagnostic agents, at most 200 diagnostic agents, at most 100 diagnostic agents.

## Patentansprüche

1. Verfahren zur Diagnose von Prostatakrebs oder des Risikos von Prostatakrebs bei einem Patienten durch Nachweisen von Antikörpern gegen die folgenden Markerproteine oder eine Auswahl von mindestens 2 der Markerproteine oder mindestens 20 % der Markerproteine, ausgewählt aus OXA1L, GOLM1, NRXN2, PAPSS1, GNAI2, FTSJD2, CERS1, FNTB, MY019, ADCK3, SDHA, FAM184A (Liste 1) bei einem Patienten, wobei die Auswahl mindestens OXA1L aufweist, aufweisend den Schritt des Nachweisens von Antikörpern, welche die Markerproteine in einer Probe des Patienten binden.

2. Verfahren zur Diagnose von Prostatakrebs oder des Risikos von Prostatakrebs bei einem Patienten durch Nachweisen von Antikörpern gegen mindestens 2 der Markerproteine oder mindestens 20 % der Markerproteine, ausgewählt aus den Markern einer der Listen 2, 3, 4 oder einer beliebigen Kombination davon bei einem Patienten, wobei die Marker mindestens OXA1L aufweisen, aufweisend den Schritt des Nachweisens von Antikörpern, welche die Markerproteine in einer Probe des Patienten binden.

3. Verfahren nach Anspruch 2, aufweisend das Nachweisen eines Antikörpers gegen ein Markerprotein, ausgewählt aus einer der Listen 5, 6, 7, 8, 9, 10, 11, 12 oder 13 bei einem Patienten, aufweisend den Schritt des Nachweisens von Antikörpern, die das Markerprotein in einer Probe des Patienten binden.

4. Verfahren nach Anspruch 2, aufweisend das Nachweisen von Antikörpern gegen mindestens 2 oder mindestens 20 % der Markerproteine, ausgewählt aus den Markern einer der Listen 5, 6, 7, 8, 9, 10, 11, 12 oder 13 bei einem Patienten, aufweisend den Schritt des Nachweisens von Antikörpern, welche die Markerproteine in einer Probe des Patienten binden.

5. Verfahren nach Anspruch 2, aufweisend das Nachweisen von Antikörpern gegen mindestens 2 oder mindestens 20 % der Markerproteine, ausgewählt aus den Markern einer der Listen 3p1, 3p2, 3p3 bei einem Patienten, aufweisend den Schritt des Nachweisens von Antikörpern, welche die Markerproteine in einer Probe des Patienten binden.

6. Verfahren nach einem der Ansprüche 1 bis 5, aufweisend das Nachweisen von mindestens den Markern SDHA und/oder FAM184A bei einem Patienten, aufweisend den Schritt des Nachweisens von Antikörpern, welche die Markerproteine in einer Probe des Patienten binden.

7. Verfahren nach einem der Ansprüche 1 bis 6, ferner aufweisend das Nachweisen von PSA in einer Probe von einem Patienten, aufweisend den Schritt des Nachweisens des Markerproteins oder antigener Fragmente davon in einer Probe des Patienten.

8. Verfahren nach Anspruch 7, wobei ein PSA-Protein in der Probe durch einen Affinitätsassay, vorzugsweise mit einem immobilisierten Affinitäts-Einfangmittel, nachgewiesen wird.

9. Verfahren nach einem der Ansprüche 1 bis 8, wobei der Schritt des Nachweisens von Antikörpern, welche die Markerproteine binden, das Vergleichen des Nachweissignals mit Nachweissignalen einer gesunden Kontrolle und Vergleichen der Nachweissignale aufweist, wobei eine Zunahme des Nachweissignals Prostatakrebs anzeigt.

10. Verfahren nach einem der Ansprüche 1 bis 9, a) wobei der Schritt des Nachweisens von Antikörpern, welche die Markerproteine binden, das Vergleichen des Nachweisignals mit Nachweissignalen von einem oder mehreren bekannten Prostatakrebs-Kontrollproben aufweist, wobei bevorzugt die Kontrollsignale verwendet werden, um ein markerabhängiges Signalmuster als Indikations-Klassifikator zu erhalten, und wobei die markerabhängigen Signale des Patienten mit dem Muster verglichen werden und/oder an dieses Muster angepasst werden, wodurch Informationen der diagnostizierten Erkrankung erhalten werden.

11. Verfahren nach einem der Ansprüche 1 bis 10, a) wobei der Schritt des Nachweisens von Antikörpern, welche die Markerproteine binden, das Vergleichen des Nachweissignals mit Nachweissignalen einer kanzerösen Kontrolle und Vergleichen der Nachweissignale aufweist, wobei ein Nachweissignal von der Probe des Patienten mit einer Amplitude von mindestens 60 %, vorzugsweise mindestens 80 %, der kanzerösen Kontrolle Prostatakrebs anzeigt; oder b) wobei ein Nachweissignal bei mindestens 60 %, vorzugsweise mindestens 75 %, der verwendeten Marker Prostatakrebs anzeigt.

12. Diagnosemittel-Kit, das zum Nachweisen von Antikörpern gegen einen Marker oder eine Markerkombination wie definiert in den Ansprüchen 1 bis 9 geeignet ist, wobei der Marker oder die Markerkombination mindestens OXA1L aufweist, wobei die Diagnosemittel Markerproteine oder antigene Fragmente davon aufweisen, die zum Binden von Antikörpern in einer Probe geeignet sind, wobei das Kit ferner ein computerlesbares Medium oder ein Computerprogrammprodukt aufweist, aufweisend Signaldaten für Kontrollproben mit bekannten Erkrankungen, ausgewählt aus Prostatakrebs, und/oder Kalibrierungs- oder Trainingsdaten zum Analysieren der Marker, die in dem Kit zum Diagnostizieren von Prostatakrebs oder zum Unterscheiden von Erkrankungen, ausgewählt aus gesunden Bedingungen und Prostatakrebs, bereitgestellt sind.

13. Das Diagnosemittel-Kit nach Anspruch 12, wobei die Diagnosemittel auf einem festen Träger immobilisiert sind.

14. Das Diagnosemittel-Kit nach Anspruch 12 oder 13, aufweisend einen markierten sekundären Antikörper, vorzugsweise zum Nachweisen eines Fc-Teils von Antikörpern des Patienten.

15. Das Diagnosemittel-Kit nach Anspruch 12, 13 oder 14, aufweisend höchstens 3 000 Diagnosemittel, vorzugsweise höchstens 2 500 Diagnosemittel, höchstens 2 000 Diagnosemittel, höchstens 1 500 Diagnosemittel, höchstens 1 200 Diagnosemittel, höchstens 1 000 Diagnosemittel, höchstens 800 Diagnosemittel, höchstens 500 Diagnosemittel, höchstens 300 Diagnosemittel, höchstens 200 Diagnosemittel, höchstens 100 Diagnosemittel.

## Revendications

1. Procédé destiné à diagnostiquer un cancer de la prostate ou un risque de cancer de la prostate chez un patient en détectant des anticorps dirigés contre les protéines marqueurs suivantes, ou contre une sélection d'au moins 2 des protéines marqueurs, ou contre au moins 20 % des protéines marqueurs sélectionnées parmi OXA1L, GOLM1, NRXN2, PAPSS1, GNA12, FTSJD2, CERS1, FNTB, MYO19, ADCK3, SDHA, FAM184A (Liste 1) chez un patient, où la sélection comprend au moins OXA1L, comprenant une étape de détection des anticorps qui se lient auxdites protéines marqueurs dans un échantillon du patient.

2. Procédé destiné à diagnostiquer un cancer de la prostate ou un risque de cancer de la prostate chez un patient en détectant des anticorps dirigés contre au moins 2 des protéines marqueurs, ou contre au moins 20 % des protéines marqueurs sélectionnées parmi les marqueurs de n'importe laquelle des listes 2, 3, 4 ou de toute combinaison de celles-ci, chez un patient, où les marqueurs comprennent au moins OXA1L, comprenant une étape de détection des anticorps qui se lient auxdites protéines marqueurs dans un échantillon du patient.

3. Procédé selon la revendication 2, comprenant une étape de détection d'un anticorps dirigé contre une protéine marqueur sélectionnée parmi l'une quelconque des listes 5, 6, 7, 8, 9, 10, 11, 12 ou 13, chez un patient, comprenant une étape de détection des anticorps qui se lient à ladite protéine marqueur dans un échantillon du patient.

4. Procédé selon la revendication 2, comprenant une étape de détection des anticorps dirigés contre au moins 2 ou au moins 20 % des protéines marqueurs sélectionnées parmi les marqueurs de l'une quelconque des listes 5, 6, 7, 8, 9, 10, 11, 12 ou 13, chez un patient, comprenant une étape de détection des anticorps qui se lient auxdites protéines marqueurs dans un échantillon du patient.

5. Procédé selon la revendication 2, comprenant une étape de détection des anticorps dirigés contre au moins 2 ou au moins 20 % des protéines marqueurs sélectionnées parmi les marqueurs de l'une quelconque des listes 3p1, 3p2, 3p3, chez un patient, comprenant une étape de détection des anticorps qui se lient auxdites protéines marqueurs dans un échantillon du patient.

6. Procédé selon l'une quelconque des revendications 1 à 5, comprenant la détection au moins des marqueurs SDHA et/ou FAM184A chez un patient, comprenant une étape de détection des anticorps qui se lient auxdites protéines marqueurs dans un échantillon du patient.

7. Procédé selon l'une quelconque des revendications 1 à 6, comprenant en outre la détection de PSA dans un échantillon d'un patient comprenant une étape de détection de ladite protéine marqueur ou des fragments antigéniques de celle-ci dans un échantillon du patient.

8. Procédé selon la revendication 7, où la protéine PSA dans l'échantillon est détectée par un test d'affinité, de préférence avec un agent de capture d'affinité immobilisé.

9. Procédé selon l'une quelconque des revendications 1 à 8, dans lequel l'étape de détection des anticorps qui se lient auxdites protéines marqueurs, comprend la comparaison dudit signal de détection avec des signaux de détection d'un échantillon témoin sain, et la comparaison desdits signaux de détection, où une augmentation du signal de détection indique un cancer de la prostate.

10. Procédé selon l'une quelconque des revendications 1 à 9, a) où l'étape de détection des anticorps qui se lient auxdites protéines marqueurs, comprend une étape de comparaison dudit signal de détection à des signaux de détection d'un ou de plusieurs échantillons témoins connus de cancer de la prostate, de préférence où les signaux de contrôle sont utilisés afin d'obtenir un motif de signal dépendant du marqueur en tant que d'indication de classement, et les signaux dépendants du marqueur du patient, sont comparés audit motif et/ou mis en correspondance avec celui-ci, obtenant de ce fait une information sur la maladie diagnostiquée.

11. Procédé selon l'une quelconque des revendications 1 à 10, a) où l'étape de détection des anticorps qui se lient auxdites protéines marqueurs, comprend la comparaison dudit signal de détection à des signaux de détection d'un échantillon témoin cancéreux, et à comparer lesdits signaux de détection, où un signal de détection de l'échantillon du patient dont l'amplitude est au moins égale à 60 %, de préférence au moins égale à 80 %, de l'échantillon témoin cancéreux, indique un cancer de la prostate ; ou b) où un signal de détection d'au moins 60 %, de préférence au moins 75 %, des marqueurs utilisés, indique un cancer de la prostate.

12. Kit d'agents de diagnostique appropriés à une détection d'anticorps dirigés contre tout marqueur ou combinaison de marqueurs tels que définis dans les revendications 1 à 9, où le marqueur ou la combinaison de marqueurs, comprend au moins OXA1L, où lesdits agents de diagnostiques comprennent des protéines marqueurs ou des fragments antigéniques de celles-ci permettant la liaison à des anticorps dans un échantillon, le kit comprenant en outre un support pouvant être lu par un ordinateur, ou un produit programme informatique, comprenant des données de signal d'échantillons témoins dans des conditions connues sélectionnées parmi un cancer de la prostate, et/ou des données d'étalonnage ou d'apprentissage destinées à analyser lesdits marqueurs fournis dans le kit afin de diagnostiquer un cancer de la prostate, ou de distinguer des états sélectionnées parmi des états sains et un cancer de la prostate.

13. Le kit selon la revendication 12, où lesdits agents de diagnostiques sont immobilisés sur un support solide.

14. Le kit selon la revendication 12 ou 13, comprenant un anticorps secondaire marqué, de préférence afin de détecter la partie Fc d'anticorps du patient.

15. Le kit selon la revendication 12, 13 ou 14, comprenant tout au plus 3000 agents de diagnostiques, de préférence tout au plus 2500 agents de diagnostiques, tout au plus 2000 agents de diagnostiques, tout au plus 1500 agents de diagnostiques, tout au plus 1200 agents de diagnostiques, tout au plus 1000 de agents diagnostiques, tout au plus 800 agents de diagnostiques, tout au plus 500 agents de diagnostiques, tout au plus 300 agents de diagnostiques, tout au plus 200 agents de diagnostiques, tout au plus 100 agents de diagnostiques.
